(19) Europäisches Patentamt
European Patent Office
Office européen des brevets

(11) EP 1 667 044 A2

(12) EUROPEAN PATENT APPLICATION

(43) Date of publication:
**07.06.2006 Bulletin 2006/23**

(51) Int Cl.:
***G06F 19/00*** *(2006.01)*

(21) Application number: **05025675.9**

(22) Date of filing: **24.11.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **06.12.2004 JP 2004353068**

(71) Applicant: **HITACHI SOFTWARE ENGINEERING CO., LTD.**
**Yokohama-shi, Kanagawa 230-0045 (JP)**

(72) Inventors:
- **Matsumoto, Toshiko**
  **Tokyo 140-0002 (JP)**
- **Yukawa, Wataru**
  **Tokyo 140-0002 (JP)**
- **Nakashige, Ryo**
  **Tokyo 140-0002 (JP)**

(74) Representative: **Liesegang, Roland**
**FORRESTER & BOEHMERT**
**Pettenkoferstrasse 20-22**
**80336 München (DE)**

(54) **Method and apparatus for displaying gene information**

(57) A method and apparatus for estimating the relative height of a stutter peak in an individual typing experiment and a pooled typing experiment which is conducted on a DNA marker with high accuracy without performing an additional preliminary experiment, and displaying the result of the experiment or the like that has been corrected in accordance with the results of estimation. A determination as to whether or not a DNA marker is a compound marker and the estimation of the relative height of a stutter peak are made by utilizing the intervals of peaks in a waveform of a detection signal obtained from a PCR amplification product and the features of published genome sequences.

FIG. 1

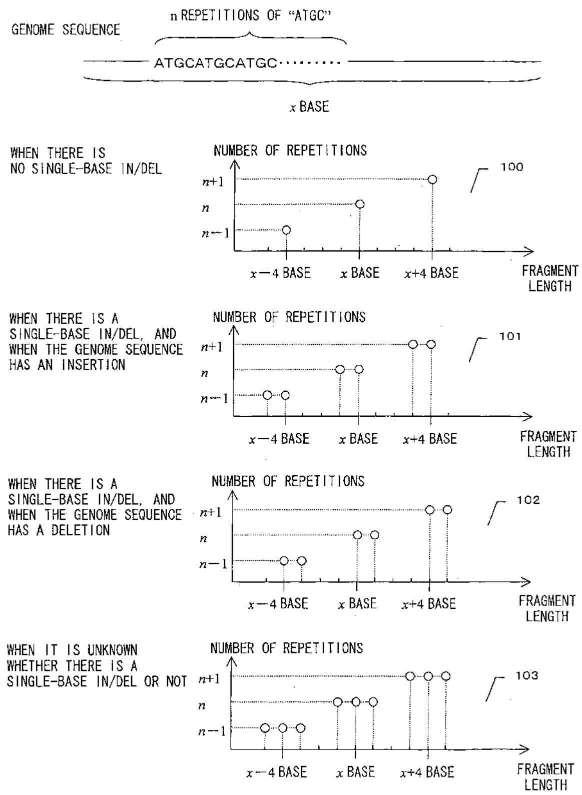


EP 1 667 044 A2

SEQUENCE LISTING

<110> Hitachi Software Engineering Co., Ltd.

<120> METHOD AND APPARATUS FOR DISPLAYING GENE INFORMATION

<130> PH-2595

<150> JP2004-353068
<151> 2004-12-06

<160> 54

<170> PatentIn Ver. 2.1

<210> 1
<211> 12
<212> DNA
<213> Artificial Sequence

<220>
<223> Description of Artificial Sequence:Synthetic DNA

<400> 1
atgcatgcat gc 12

<210> 2
<211> 80
<212> DNA

<213> Artificial Sequence

<220>

<223> Description of Artificial Sequence:Synthetic DNA

<400> 2

atgcatgcat ggatggatga tgatgatggc atgcttgttg ttgttgatgc gtgtgtatgg 60

atggatggat ggatgcatgg 80

<210> 3

<211> 10

<212> DNA

<213> Artificial Sequence

<220>

<223> Description of Artificial Sequence:Synthetic DNA

<400> 3

atatatatat 10

<210> 4

<211> 14

<212> DNA

<213> Artificial Sequence

<220>

<223> Description of Artificial Sequence:Synthetic DNA

<400> 4

atatatatat atat 14

<210> 5

<211> 12

<212> DNA

<213> Artificial Sequence

<220>

<223> Description of Artificial Sequence:Synthetic DNA

<400> 5

atatatatat at 12

<210> 6

<211> 12

<212> DNA

<213> Artificial Sequence

<220>

<223> Description of Artificial Sequence:Synthetic DNA

<400> 6

atatatatat at 12

<210> 7

<211> 66

<212> DNA

<213> Artificial Sequence

<220>

<223> Description of Artificial Sequence:Synthetic DNA

<400> 7

atgcgactat gcgactgcgc ctatatatat atatatatat atatatcggc tacgagctta 60

cgagct 66

<210> 8

<211> 58

<212> DNA

<213> Artificial Sequence

<220>

<223> Description of Artificial Sequence:Synthetic DNA

<400> 8

atgcgactat gcgactgcgc ctatatatat atatatatcg gctacgagct tacgagct 58

<210> 9

<211> 66

<212> DNA

<213> Artificial Sequence

<220>

<223> Description of Artificial Sequence:Synthetic DNA

<400> 9

atgcgactat gcgactgcgc ctatatatat atatatatat atatatcggc tacgagctta 60

cgagct 66

<210> 10

<211> 66

<212> DNA

<213> Artificial Sequence

<220>

<223> Description of Artificial Sequence:Synthetic DNA

<400> 10

atgcgactat gcgactgcgc ctatatatat atatatatat atatatcggc tacgagctta 60

cgagct 66

<210> 11

<211> 58

<212> DNA

<213> Artificial Sequence

<220>

<223> Description of Artificial Sequence:Synthetic DNA

<400> 11

atgcgactat gcgactgcgc ctatatatat atatatatcg gctacgagct tacgagct 58

<210> 12

<211> 58

<212> DNA

<213> Artificial Sequence

<220>

<223> Description of Artificial Sequence:Synthetic DNA

<400> 12

atgcgactat gcgactgcgc ctatatatat atatatatcg gctacgagct tacgagct 58

<210> 13

<211> 10

<212> DNA

<213> Artificial Sequence

<220>

<223> Description of Artificial Sequence:Synthetic DNA

<400> 13

atatatatat 10

<210> 14

<211> 14

<212> DNA

<213> Artificial Sequence

<220>

<223> Description of Artificial Sequence:Synthetic DNA

<400> 14

atatatatat atat 14

<210> 15

<211> 12

<212> DNA

<213> Artificial Sequence

<220>

<223> Description of Artificial Sequence:Synthetic DNA

<400> 15

atatatatat at 12

<210> 16

<211> 12

<212> DNA

<213> Artificial Sequence

<220>

<223> Description of Artificial Sequence:Synthetic DNA

<400> 16

atatatatat at 12

<210> 17

<211> 10

<212> DNA

<213> Artificial Sequence

<220>

<223> Description of Artificial Sequence:Synthetic DNA

<400> 17

atatatatat 10

<210> 18

<211> 12

<212> DNA

<213> Artificial Sequence

<220>

<223> Description of Artificial Sequence:Synthetic DNA

<400> 18

atatatatat at 12

<210> 19

<211> 10

<212> DNA

<213> Artificial Sequence

<220>

<223> Description of Artificial Sequence:Synthetic DNA

<400> 19

atatatatat 10

<210> 20

<211> 10

<212> DNA

<213> Artificial Sequence

<220>

<223> Description of Artificial Sequence:Synthetic DNA

<400> 20

atatatatat 10

<210> 21

<211> 12

<212> DNA

<213> Artificial Sequence

<220>

<223> Description of Artificial Sequence:Synthetic DNA

<400> 21

atatatatat at 12

<210> 22

<211> 12

<212> DNA

<213> Artificial Sequence

<220>

<223> Description of Artificial Sequence:Synthetic DNA

<400> 22

atatatatat at 12

<210> 23

<211> 12

<212> DNA

<213> Artificial Sequence

<220>

<223> Description of Artificial Sequence:Synthetic DNA

<400> 23

atatatatat at 12

<210> 24

<211> 14

<212> DNA

<213> Artificial Sequence

<220>

<223> Description of Artificial Sequence:Synthetic DNA

<400> 24

atatatatat atat 14

<210> 25

<211> 10

<212> DNA

<213> Artificial Sequence

<220>

<223> Description of Artificial Sequence:Synthetic DNA

<400> 25

atatatatat 10

<210> 26

<211> 14

<212> DNA

<213> Artificial Sequence

<220>

<223> Description of Artificial Sequence:Synthetic DNA

<400> 26

gtgtgtgtgt gtgt 14

<210> 27

<211> 14

<212> DNA

<213> Artificial Sequence

<220>

<223> Description of Artificial Sequence:Synthetic DNA

<400> 27

atatatatat atat 14

<210> 28

<211> 10

<212> DNA

<213> Artificial Sequence

<220>

<223> Description of Artificial Sequence:Synthetic DNA

<400> 28

gtgtgtgtgt 10

<210> 29

<211> 12

<212> DNA

<213> Artificial Sequence

<220>

<223> Description of Artificial Sequence:Synthetic DNA

<400> 29

atatatatat at 12

<210> 30

<211> 12

<212> DNA

<213> Artificial Sequence

<220>

<223> Description of Artificial Sequence:Synthetic DNA

<400> 30

atatatatat at 12

<210> 31

<211> 10

<212> DNA

<213> Artificial Sequence

<220>

<223> Description of Artificial Sequence:Synthetic DNA

<400> 31

gtgtgtgtgt 10

<210> 32

<211> 12

<212> DNA

<213> Artificial Sequence

<220>

<223> Description of Artificial Sequence:Synthetic DNA

<400> 32

atgcatgcat gc 12

<210> 33

<211> 16

<212> DNA

<213> Artificial Sequence

<220>

<223> Description of Artificial Sequence:Synthetic DNA

<400> 33

atgcatgcat gcatgc 16

<210> 34

<211> 66

<212> DNA

<213> Artificial Sequence

<220>

<223> Description of Artificial Sequence:Synthetic DNA

<400> 34

atgcgactat gcgactgcgc ctatatatat atatatatat atatatcggc tacgagctta 60
cgagct 66

<210> 35

<211> 66

<212> DNA

<213> Artificial Sequence

<220>

<223> Description of Artificial Sequence:Synthetic DNA

<400> 35

atgcgactat gcgactgcgc ctatatatat atatatatat atatatcggc tacgagctta 60
cgagct 66

<210> 36

<211> 66

<212> DNA

<213> Artificial Sequence

<220>

<223> Description of Artificial Sequence:Synthetic DNA

<400> 36

atgcgactat gcgactgcgc ctatatatat atatatatat atatatcggc tacgagctta 60
cgagct 66

<210> 37
<211> 64
<212> DNA
<213> Artificial Sequence

<220>
<223> Description of Artificial Sequence:Synthetic DNA

<400> 37
atgcgactat gcgactgcgc ctatatatat atatatatat atatcggcta cgagcttacg 60
agct 64

<210> 38
<211> 64
<212> DNA
<213> Artificial Sequence

<220>
<223> Description of Artificial Sequence:Synthetic DNA

<400> 38
atgcgactat gcgactgcgc ctatatatat atatatatat atatcggcta cgagcttacg 60
agct 64

<210> 39
<211> 68
<212> DNA
<213> Artificial Sequence

<220>
<223> Description of Artificial Sequence:Synthetic DNA

<400> 39
atgcgactat gcgactgcgc ctatatatat atatatatat atatatatcg gctacgagct 60
tacgagct 68

<210> 40
<211> 68
<212> DNA
<213> Artificial Sequence

<220>
<223> Description of Artificial Sequence:Synthetic DNA

<400> 40
atgcgactat gcgactgcgc ctatatatat atatatatat atatatatcg gctacgagct 60
tacgagct 68

<210> 41
<211> 10
<212> DNA
<213> Artificial Sequence

<220>

<223> Description of Artificial Sequence:Synthetic DNA

<400> 41

atatatatat 10

<210> 42

<211> 14

<212> DNA

<213> Artificial Sequence

<220>

<223> Description of Artificial Sequence:Synthetic DNA

<400> 42

atatatatat atat 14

<210> 43

<211> 12

<212> DNA

<213> Artificial Sequence

<220>

<223> Description of Artificial Sequence:Synthetic DNA

<400> 43

atatatatat at 12

<210> 44
<211> 12
<212> DNA
<213> Artificial Sequence

<220>
<223> Description of Artificial Sequence:Synthetic DNA

<400> 44
atatatatat at 12

<210> 45
<211> 10
<212> DNA
<213> Artificial Sequence

<220>
<223> Description of Artificial Sequence:Synthetic DNA

<400> 45
atatatatat 10

<210> 46
<211> 12
<212> DNA
<213> Artificial Sequence

<220>

<223> Description of Artificial Sequence:Synthetic DNA

<400> 46

atatatatat at 12

<210> 47

<211> 10

<212> DNA

<213> Artificial Sequence

<220>

<223> Description of Artificial Sequence:Synthetic DNA

<400> 47

atatatatat 10

<210> 48

<211> 10

<212> DNA

<213> Artificial Sequence

<220>

<223> Description of Artificial Sequence:Synthetic DNA

<400> 48

atatatatat 10

<210> 49
<211> 12
<212> DNA
<213> Artificial Sequence

<220>
<223> Description of Artificial Sequence:Synthetic DNA

<400> 49
atatatatat at 12

<210> 50
<211> 12
<212> DNA
<213> Artificial Sequence

<220>
<223> Description of Artificial Sequence:Synthetic DNA

<400> 50
atatatatat at 12

<210> 51
<211> 12
<212> DNA
<213> Artificial Sequence

<220>

<223> Description of Artificial Sequence:Synthetic DNA

<400> 51

atatatatat at 12

<210> 52

<211> 14

<212> DNA

<213> Artificial Sequence

<220>

<223> Description of Artificial Sequence:Synthetic DNA

<400> 52

atatatatat atat 14

<210> 53

<211> 10

<212> DNA

<213> Artificial Sequence

<220>

<223> Description of Artificial Sequence:Synthetic DNA

<400> 53

atatatatat 10

<210> 54

<211> 10

<212> DNA

<213> Artificial Sequence

<220>

<223> Description of Artificial Sequence:Synthetic DNA

<400> 54

atatatatat 10

## Description

BACKGROUND OF THE INVENTION

Field of the Invention

The present invention relates to a method and apparatus for displaying gene information used for analysis for identifying genes involved in phenotypes, such as an individual's diseases or external features. In particular, the invention relates to a method and apparatus capable of displaying the results of analysis in which, when extracting and detecting a DNA fragment with a gene as the subject of analysis using PCR or electrophoresis, a signal from the subject of analysis and noise signals are clearly distinguished, and corrections are made so as to eliminate the influence of the noise signals on the desired signal.

Background Art

Following the completion of the sequencing of the human genome, research is actively underway so as to analyze the function of genes. Among other factors, particular attention is being focused on the automatic determination of genotypes and genotype frequency, which form the basis for a search for genes involved in phenotypes, such as the presence or absence of particular diseases, the extent of efficacy of medication, and the presence or absence of side effects.

Microsatellite

Normally, genomes of living organisms of the same species have substantially identical nucleotide sequences, with different nucleotides located at some sites. For example, at a certain genetic locus, some individuals may have A while other individuals may have T. Such presence of polymorphism in a single nucleotide of a genome among individuals is referred to as a SNP (Single Nucleotide Polymorphism).

There are other cases where one individual has A at a certain genetic locus and other individuals do not. For example, as shown in Fig. 12, the genome of individual A has a single nucleotide A between the nucleotide sequence "NNNNNNNNNN" and the nucleotide sequence "MMMMMMMMMMM," while the genome of another individual B does not have such a nucleotide. (In the drawings, "NNNNNNNNN" and "MMMMMMMMMMM" each represent arbitrary nucleotide sequences). In this case, the genome of individual B lacks the single nucleotide A from the viewpoint of individual A. However, from the viewpoint of individual B, the single nucleotide A is inserted in the genome of individual A. Such polymorphism based on a difference in terms of the presence or absence of a single nucleotide at a single genetic locus between individuals is referred to as "in/del" (short for insertion/deletion) of a single nucleotide.

The genomes of living organisms have many (tens of thousands or more) sites at which a short nucleotide sequence pattern that is two to six nucleotides long appears repeatedly several to a dozen times. Such a characteristic nucleotide sequence pattern is referred to as a microsatellite. An example of microsatellite that appears in a genome is shown in Fig. 13. A single repetition of a microsatellite is referred to as a unit, and the number of nucleotides in a unit is referred to as the unit length. For example, in the case of the microsatellite ATATATAT... shown in Fig. 13, the unit is "AT," and the unit length is two nucleotides. As shown in Fig. 13, the number of repetitions in a microsatellite may differ from one individual to another even if the individuals share the same unit and the unit length. In the following, a microsatellite regarding which the number of repetitions is known to vary among individuals is referred to as "a microsatellite with polymorphism," and a microsatellite regarding which the number of repetitions is known to be the same in all individuals will be referred to as "a microsatellite without polymorphism." A microsatellite regarding which it is not known whether the number of repetitions differs or not among individuals will be referred to as "a microsatellite regarding which the presence or absence of polymorphism is unknown."

As described above, SNPs, single nucleotide in/del, and microsatellites, which can vary among individuals, are portions that can be easily distinguished from other nucleotide sequences in a genome, and they can also be easily detected experimentally. In some species of living organisms, the approximate positions of SNPs, single-nucleotide in/del, and microsatellites in the genome are known, and therefore they can be used as indices of genomic positions. Because of these characteristics, SNPs, single-nucleotide in/del's, and microsatellites with polymorphisms are referred to as DNA markers. In particular, microsatellites with polymorphisms, which include a plurality of nucleotides, contain much more amount of information than SNPs or single-nucleotide in/del's, and therefore they are used frequently as DNA markers. Further, microsatellites with polymorphism have an added advantage that a plurality of samples can be subjected to experimentation simultaneously in a pooled typing experiment, as will be described later.

As shown in Figs. 12 and 13, individuals of many different species have a pair of genomes (homologous chromosomes) that are derived from a female gamete and a male gamete. Genes that exist at corresponding sites on a set of genomes are referred to as alleles, and their combinations are referred to as genotypes. As mentioned above, SNPs, single-nucleotide in/del's, and microsatellites with polymorphisms constitute portions that can contain different nucleotide sequences among individuals. Therefore, generally two to three alleles exist for an SNP, two alleles exist for a single-nucleotide in/del, and several to 20 or more kinds of alleles exist for a microsatellite with polymorphism.

In the example shown in Fig. 13, individual A has a microsatellite consisting of five repetitions of the "AT" unit, and a microsatellite consisting of seven repetitions of such unit. Individual B, on the other hand, has two microsatellites each consisting of six "AT" units. In this case, the condition where the individual has two alleles of different kinds, as in the case of individual A, is denoted by the term "heterozygous", while the condition where the individual has two alleles of the same kind, as in individual B, is denoted by the term "homozygous."

PCR, electrophoresis experiment, and pooled typing experiment

When a microsatellite with polymorphism is used as a DNA marker, an experiment such as PCR (Polymerase Chain Reaction) or electrophoresis is carried out to extract and detect the sites in the genome where microsatellites appear. PCR is an experimental technique whereby a pair of nucleotide sequences called primer sequences are designated at either end of a microsatellite, and then only those portions between the thus designated nucleotide sequences are repeatedly replicated as DNA fragments so as to obtain a predetermined amount of a sample. Electrophoresis, examples of which include gel electrophoresis and capillary electrophoresis, is an experimental technique involving causing an amplified DNA fragment to electrophorese in an electrically charged migration path so as to separate DNA fragments with different lengths. Thus, electrophoresis is a sample separation technique that takes advantage of the difference in migration speeds in a migration path depending on the length of DNA fragments (the longer the DNA fragment, the smaller its migration speed).

Fig. 14 schematically shows experimental procedures for extracting and amplifying DNA fragments in microsatellites using PCR and gel electrophoresis. First, a pair of primer sequences 1400 and 1401 at either end of a target microsatellite are designated, and then a genome region 1402 including the microsatellite and the primer sequences is amplified in a PCR experiment. The example shown in Fig. 14 is heterozygous, in which the number of repetitions of a microsatellite in each of the two homologous chromosomes is different. Because the lengths of the microsatellites are different, two kinds of PCR amplification products, namely, DNA fragments, that have different lengths (66 nucleotides and 58 nucleotides) are obtained from each allele. When these DNA fragments are subjected to electrophoresis on slab gel for a predetermined time, the two kinds of PCR amplification products are separated, depending on the difference in the length of the DNA fragments. Each of the DNA fragments is labeled with a fluorescent dye in advance. As shown in Fig. 14, after electrophoresis is completed, the intensity and position of fluorescent signals from each DNA fragment are detected, based on which a graph can be plotted in which the horizontal axis shows the length of DNA fragments (i.e., the distance of migration) and the vertical axis shows the intensity of fluorescent signals (i.e., the number of DNA fragments that are present). Together with the PCR amplification products, a DNA fragment with a known length (referred to as a size maker) can also be subjected to electrophoresis and a fluorescent signal from it can be detected. In this way, the length of each PCR amplification product can also be determined with reference to the position where the size marker is detected.

While an experimental technique involving gel electrophoresis has been described above, the same procedure can be performed for capillary electrophoresis. In capillary electrophoresis, samples are caused to migration over a thin tube filled with gel, and the time it takes for each sample to complete migrating a predetermined distance (normally to the end of the capillary) is measured so as to determine the length of the DNA fragments. In capillary electrophoresis, instead of scanning the samples in gel for fluorescent signals, samples are generally detected using a fluorescent signal detector fitted at the end of the capillary.

The experiment performed on a sample from a single individual involving PCR and electrophoresis, as in Fig. 14, is referred to as an individual typing experiment. And the graph obtained from such an individual typing experiment, in which the horizontal axis shows the length of DNA fragments and the vertical axis shows fluorescent signal intensities, is referred to as an individual typing waveform. On the other hand, an experiment performed on samples from a plurality of individuals in a single batch involving PCR and electrophoresis is referred to as a pooled typing experiment. The graph obtained from a pooled typing experiment (which is plotted in the same way as mentioned above) is referred to as a pooled typing waveform. The pooled typing experiment allows the frequency distribution of alleles in samples from a plurality of individuals to be measured in a single experiment.

Fig. 15 schematically shows a procedure for a pooled typing experiment in which PCR and electrophoresis are carried out for samples from a plurality of individuals in a single batch. A sample DNA fragment of individual A is a heterozygote comprised of 58 nucleotides and 62 nucleotides. A sample DNA fragment of individual B is a homozygote comprised of 60 nucleotides. A sample DNA fragment of individual C is a heterozygote comprised of 58 nucleotides and 60 nucleotides. Equal amounts of samples are collected from individuals A to C and mixed, thereby preparing a pooled sample. This pooled sample includes the alleles of 58 nucleotides derived from individuals A and C, alleles of 60 nucleotides derived from individuals B and C, and an allele of 62 nucleotides derived from individual A. Because the sample DNA fragment of individual B is a homozygote comprised of alleles of 60 nucleotides, the amount of the allele of 60 nucleotides derived from individual B that is included in the pooled sample is twice as much as the amount of the allele of 60 nucleotides derived from individual C. During the amplification by PCR and the detection of fluorescent signals by electrophoresis, the aforementioned ratio of amounts is substantially unchanged. Therefore, when a waveform of the pooled sample is obtained using PCR and electrophoresis, the heights of the peaks that appear at the location of each nucleotide length are substantially proportional to the amount of the allele of the corresponding nucleotide length that is present in the pooled sample. In the example of Fig. 15, the percentage of the height of each peak to the sum of the heights of the heights of the peaks is 33.2% for the peak of the allele of 58 nucleotides, 50.0% for the peak of 60 nucleotides, and 16.7% for the allele of 62 nucleotides. Since samples from three individuals were mixed, the total of the allele frequencies is 2 x 3 = 6. Therefore, the frequency of appearance of each allele in the pooled sample can be calculated. Because the pooled typing experiment requires that PCR and electrophoresis be performed only once each, costs and time required for the experiment can be greatly reduced as compared with a case where individual typing experiments are repeated as many times as there are samples.

Phenomena during an actual experiment

The aforementioned experimental results shown in Figs. 14 and 15 would be obtained when the PCR and electrophoresis experiments were to be performed through an ideal process and, in addition, on the assumption of a DNA marker that would exhibit a simple polymorphism. In reality, however, various forms of noise can be produced during an experiment or polymorphisms may be present in a combined manner. Thus, in the following, a stutter peak, which is a typical noise peak produced during the PCR and electrophoresis experiments, and polymorphisms that are produced in a combined manner will be described with reference to examples.

Gene that produces complex polymorphism

The DNA marker in which a single-nucleotide in/del or a microsatellite with polymorphism appears in a combined manner is referred to as a compound marker. In a compound marker, complex polymorphisms are observed, such as an instance of polymorphism where, when microsatellites with the same number of repetitions exist, the fragment lengths are not necessarily the same.

Fig. 16 shows DNA fragments containing two kinds of microsatellites with polymorphism as an example of a compound marker. In these DNA fragments, the unit lengths or the number of repetitions of a unit could differ between the individual microsatellites. As a result, the DNA fragments, even if they have the same length, could have different nucleotide sequences. For example, the DNA fragment of individual A includes a microsatellite consisting of five repetitions of "AT" and a microsatellite consisting of seven repetitions of "GT" in one allele, and a microsatellite consisting of seven repetitions of "AT" and a microsatellite consisting of five repetitions of "GT" in the other allele. These two alleles have the same nucleotide length because the sum of the lengths of the two microsatellites in each allele is the same, but the nucleotide sequences are different.

Fig. 17 shows DNA fragments containing a microsatellite with polymorphism and a single-nucleotide in/del, as another example of the compound marker. In these DNA fragments, in addition to the possibility that the number of repetitions of the unit in the microsatellite could be different, a single-nucleotide in/del exists, so that the nucleotide length of each allele can be different in various ways. Therefore, it is often the case that in the results of PCR and electrophoresis experiments, peaks do not appear in an orderly manner at unit-length intervals.

Generally, there is no way of knowing whether or not a particular DNA fragment is a compound marker, or, if so, what the polymorphism contained in it is like, unless a DNA sequencing experiment is conducted. While the human genome has already been sequenced and made public, the published human genome information does not provide polymorphism information by itself. Further, although many polymorphisms have been reported in papers, many of them are merely based on PCR and electrophoresis experiments and stop short of DNA sequencing experiments; they simply state that "the fragment lengths differ from one individual to another." Very few of such papers report that a particular DNA fragment is a compound marker.

Noises produced in PCR and electrophoresis experiments

Fig. 18 schematically shows a stutter peak, which is a typical form of noise caused during the process of PCR and electrophoresis experiments. For simplicity's sake, Fig. 18 shows only the DNA fragment of 66 nucleotides (which contains a microsatellite in which "TA" is repeated 12 times) shown in Fig. 14 as an example. The stutter peak denotes noise caused by a phenomenon in which the number of repetitions in the microsatellite portion of a DNA fragment to be replicated increases or decreases due to slipped-strand mispairing during a PCR reaction. It is observed as a noise peak of a DNA fragment of which the number of repetitions has been increased or decreased during fluorescence analysis following electrophoresis. As shown in Fig. 18, in addition to a DNA fragment 1800 that contains a normal microsatellite in which "TA" is repeated 12 times, DNA fragments 1801 or 1802 containing abnormal microsatellites in which "TA" is repeated 11 or 13 times are produced, and they are observed in the form of stutter peaks in fluorescence analysis. Such an increase or decrease in the number of repetitions could occur to even a greater degree, so that there is a possibility that, in addition to the DNA fragment (66 nucleotides) of the same length as that of the original DNA fragment, DNA fragments whose length has been increased or decreased by an integer multiple of the unit length of the microsatellite could be produced.

The aforementioned stutter peak becomes an issue when examining the genotype of an individual via an individual typing experiment, or when examining the frequency distribution of alleles in a group of samples via a pooled typing experiment. In an individual typing experiment, a peak that appears at the position corresponding to the nucleotide length of the original DNA fragment (to be hereafter referred to as "a true peak"), which should be observed, must be distinguished from a stutter peak, so that the true peak alone can be adopted as information indicating the genotype of the individual. On the other hand, in a pooled typing experiment, a stutter peak caused by a single allele influences the height of the peaks of the surrounding alleles, which leads to the problem that the results obtained do not reflect the true frequency distribution of the allele.

With reference to Fig. 19, the aforementioned problem in the pooled typing experiment is described. An experiment is assumed in which samples from individual A (a heterozygote of 58 nucleotides and 62 nucleotides), individual B (a homozygote of 60 nucleotides), and individual C (a heterozygote of 58 nucleotides and 60 nucleotides) shown in Fig. 15 are used. Equal amounts of the samples are collected from the individuals and mixed, thereby preparing a pooled sample. When amplifying the pooled sample by PCR, in addition to the allele of 58 nucleotides and the allele of 62 nucleotides contained in individual A, DNA fragments of 56 nucleotides, 60 nucleotides, and 64 nucleotides, for example, appear due to slipped-strand mispairing. Similarly, DNA fragments of nucleotide lengths that are different from an original nucleotide length appear from the DNA fragments contained in individuals B or C due to slipped-strand mispairing. The waveform pattern obtained by electrophoresis is influenced by those fragments caused by slipped-strand mispairing, resulting in an frequency distribution that does not reflect the true frequency distribution of the alleles shown in Fig. 15 (33.2% for the 58 nucleotides, 50.0% for the 60 nucleotides, and 16.7% for the 62 nucleotides). A similar problem is evident in Fig. 20, in which the amplification of a pooled sample is influenced by a combination of the variation in the number of repetitions of microsatellites and the inclusion of a single-nucleotide in/del in the DNA fragment.

In both individual typing and pooled typing, it is important to eliminate the influence of stutter peaks as accurate experiment results are to be obtained. Therefore, characteristics of stutter peaks have been widely studied, and the following properties are now known:

- <u>Property 1:</u> When the DNA marker, individuals (alleles), and method of experiment are the same, the relative heights of stutter peaks are approximately the same over a plurality of experiments (see Non-patent Document 1).
- <u>Property 2:</u> When attention is focused on a single DNA marker and a single individual, the stutter peak is lower than the true peak, and the height of the stutter peak becomes lower as the stutter peak moves away from the true peak (see Non-patent Document 2).
- <u>Property 3</u>: When attention is focused on a single DNA marker, there is a linear relationship between the number of repetitions of a unit in a microsatellite and the relative height of the stutter peak (height of stutter peak divided by height of true peak), and the line representing this linear relationship is common to all DNA markers as long as the DNA marker is comprised of a repetition of two nucleotides (see Non-patent Document 3).

Patent Document 1 : JP Patent Application No. 2004-192559
Patent Document 2: JP Patent Application No. 2004-262431
Non-patent Document 1: Perlin, M. W., et al., "Toward Fully Automated Genotyping: Allele Assignment, Pedigree Construction, Phase Determination, and Recombination Detection in Duchenne Muscular Dystrophy," Am. J. Hum. Genet. 55, 1994, pp.777-787
Non-patent Document 2: Perlin, M. W., et al., "Toward Fully Automated Genotyping: Genotyping Microsatellite Markers by Deconvolution," Am. J. Hum. Genet. 57, 1995, pp.1199-1210
Non-patent Document 3: Lipkin, E., et al., "Quantitative Trait Locus Mapping in Dairy Cattle by Means of Selective Milk DNA Pooling Using Dinucleotide Microsatellite Markers: Analysis of Milk Protein Percentage," Genetics 149, July 1998, pp.1557-1567

## SUMMARY OF THE INVENTION

For both the individual typing experiment and the pooled typing experiment, methods for correcting experimental results taking advantage of properties 1 to 3 have been proposed. In these existing correction methods, a preliminary experiment is conducted in the preparatory stage of correction so as to determine, for a particular DNA marker, a formula for estimating the relative height of a stutter peak based on the length of a fragment. Thereafter, in the case of correction for an individual typing experiment, the true peak is identified by taking into consideration the relative height of each peak. In the case of correction for a pooled typing experiment, a correction process is performed whereby components derived from a stutter peak are subtracted from the waveform that is observed. For the determination of the aforementioned formula for determining the relative height of a stutter peak based on the length of a fragment, the following two methods have been proposed.

In one method, a DNA sequencing experiment is conducted on several to dozens of sample DNA markers so as to determine whether any of the markers is a compound marker, what the polymorphism of the compound marker is like if such marker is indeed a compound marker, and whether or not a microsatellite with polymorphism or a single-nucleotide in/del is included in the compound marker. Because the length of the fragment and the number of repetitions of a unit can be determined for each DNA marker based on the DNA sequencing experiment, the formula for estimating the relative height of a stutter peak based on the length of a fragment can be determined from a line representing the linear relationship between the number of repetitions of a unit and the relative height of a stutter peak that is common to all the DNA markers, and the relationship between the fragment length and the number of repetitions that has been determined by the DNA sequencing experiment.

The other method involves directly determining the relationship between the fragment length of a DNA marker and the relative height of a stutter peak based on an individual typing experiment for each of several to dozens of sample DNA markers. In order to determine the relative height of a stutter peak from a waveform obtained by an individual typing experiment for a particular individual, it is necessary to isolate the true peak and stutter peaks derived from the true peak from other noise peaks. However, in the case of a DNA marker that is heterozygous, two true peaks can appear in close proximity in some cases. In such cases, the resultant waveform is comprised of a complex superposition of the true peak, stutter peaks, and other noise peaks, which cannot be properly isolated. In view of this, it is necessary to prepare a large number of individuals for which individual typing experiments are conducted.

Generally, when correcting the experimental results of an individual typing experiment, the second method is employed. This is due to the fact that the second method can utilize the experimental results obtained by an individual typing experiment that has already been conducted, and that the fact that there is no need to perform an additional preliminary experiment. However, if many heterozygotes in which two true peaks appear in close proximity are included in the sample DNA markers used in an individual typing experiment, a problem arises that the relative height of a stutter peak cannot be estimated with sufficient accuracy due to the above-described reasons.

On the other hand, in a pooled typing experiment, both the first and the second methods are employed. However, as opposed to the case of the individual typing experiment, it is necessary to perform a preliminary experiment (a DNA sequencing experiment or an individual typing experiment) in addition to the pooled typing experiment regardless of which of the two methods is employed.

Thus, it is an object of the invention, which relates to a method and apparatus for displaying the results of the extraction and analysis of a DNA marker including a microsatellite with polymorphism or a single-nucleotide in/del via PCR and electrophoresis experiments, to provide a method and apparatus whereby experimental results that have already been obtained can be utilized in estimating the relative height of a stutter peak with high accuracy in both an individual typing experiment and an pooled typing experiment without the need to perform an additional preliminary experiment, and whereby experimental results can be displayed in which the influence of stutter peaks has been eliminated on the basis of the results of estimation.

With a view to achieving the foregoing object, the inventors conducted research and analysis on compound markers and have obtained the following information concerning regularity.

<u>Information 1:</u> As a result of progress in research into the gene sequences polymorphisms, several DNA markers have been analyzed to determine whether or not they are compound markers and, if so, what the polymorphisms they contain are like, and the relevant information is being accumulated in public databases.

<u>Information 2:</u> Whether or not a single-nucleotide in/del exists can be judged from waveforms that can be obtained from a pooled typing experiment or an individual typing experiment, without performing a DNA sequencing experiment. This is due to the fact that, in a waveform that is obtained from a DNA marker that includes only a microsatellite and that does not include a single-nucleotide in/del, peaks appear at unit-length intervals of a microsatellite, as shown in Fig. 19, whereas in a waveform obtained from a DNA marker that includes a single-nucleotide in/del as well, peaks appear at single-nucleotide intervals or (unit length -1)-nucleotide intervals as well, as shown in Fig. 20.

<u>Information 3:</u> It can be said empirically that the greater the number of repetitions, the more highly polymorphic the marker. For example, when a microsatellite in which a unit is repeated five times is compared with a microsatellite in which a unit is repeated 20 times, it is empirically known that the latter exhibits greater variety of polymorphisms.

Information 4: There are more DNA markers that are known not to be compound markers than DNA markers that are known to be compound markers. It can be expected, therefore, that of the DNA markers that are not yet known to be either compound markers or not compound markers, there are more DNA markers that are not compound markers than DNA markers that are compound markers.

Information 5: Even for compound markers that include single-nucleotide in/del's, the number of repetitions of microsatellites can be uniquely calculated from the fragment length of the PCR amplification product if the unit length of the microsatellite is 3 nucleotides or longer. An example of a method for such calculation is shown in Fig. 1, with reference to which a PCR amplification product of a DNA marker (with a fragment length of x nucleotides) in the published human genome sequence that includes a microsatellite in which a unit "ATGC" is repeated n times is analyzed. When this DNA marker does not include a single-nucleotide in/del, the fragment length of the PCR amplification product is x nucleotides, or the number of nucleotides x from which an integral multiple of the unit length is subtracted or to which such integral multiple is added. The relationship between the fragment length of the PCR amplification product and the number of repetitions of the unit is as shown in graph 100.
Meanwhile, when the DNA marker includes a single-nucleotide in/del and when the published human genome sequence includes a single nucleotide insertion, the fragment length of the PCR amplification product would be either x nucleotides, the number of nucleotides x from which an integral multiple of the unit length is subtracted or to which such integral multiple is added, or, possibly, such numbers of nucleotides from which 1 has been subtracted. The relationship between the fragment length of the PCR amplification product and the number of repetitions of the unit in such cases would be as shown in graph 101. On the contrary, when the DNA marker includes a single-nucleotide in/del and when the published human genome sequence includes a single nucleotide deletion, the fragment length of the PCR amplification product could be x nucleotides, the number of nucleotides x from which an integral multiple of the unit length is subtracted or to which such an integral multiple is added, or, possibly, such numbers of nucleotides to which 1 has been added. The relationship between the fragment length of the PCR amplification product and the number of repetitions of the unit in such cases would be as shown in graph 102. Further, when the DNA marker includes a single-nucleotide in/del but it is not known whether it is an insertion or deletion, the relationship between the fragment length of the PCR amplification product and the number of repetitions of the unit can be predicted to be within the range shown by graph 103. Thus, even for a compound marker that includes a single-nucleotide in/del, a linear relationship between the fragment length of the PCR amplification product and the number of repetitions of the unit can be drawn from the result of an electrophoresis experiment as long as the unit length of the microsatellite is 3 nucleotides or longer.

Information 6: For a compound marker that includes a plurality of kinds of microsatellites with the same lengths (including a case where two or more microsatellites have polymorphisms), the relative height of a stutter peak can be calculated by taking advantage of property 3 even if the nucleotide sequence of each individual is not known. For example, assume a case where it has been found that a DNA marker of interest includes two microsatellites whose unit lengths are 2 nucleotides, for which it is not known whether or not the microsatellites have polymorphism, that the first microsatellite from the published human genome sequence is repeated n1 times, that the second microsatellite is repeated n2 times, and that the length of the original DNA marker before amplification is x nucleotides. The linear relationship mentioned with reference to property 3 is assumed to be r = a x m + b where r is the relative height of a stutter peak, m is the number of repetitions of the unit, and a and b are the slope and the intercept, respectively, of the line. Under these assumptions, when the length of a certain PCR amplification product is x + 2 x n nucleotides, a relationship n1 + n2 + n = n1' +. n2' holds for the number of repetitions n1' of the first microsatellite and for the number of repetitions n2' of the second microsatellite in the PCR amplification product. Accordingly, the relative height of a stutter peak in this allele can be calculated as follows:

$$(a \times n1' + b) + (a \times n2' + b)$$

$$= a \times (n1' + n2') + 2b$$

$$= a \times (n1 + n2 + n) + 2b$$

Namely, in compound markers that include a plurality of kinds of microsatellites with the same unit lengths, even if it cannot be determined how the number of repetitions of each microsatellite has been increased or decreased (namely, the values of n1' and n2') by PCR amplification, the relative height of a stutter peak can be calculated for a particular nucleotide length if only information is available that the sample DNA that has been subjected to PCR amplification is a sample DNA of the published human genome sequence that has been increased or decreased by (unit length x n) nucleotides. This means that the relative height of a stutter peak can be calculated without the need to examine the nucleotide sequence of the PCR amplification product.

In view of the above information, the inventors have come to the conclusion that, in order to estimate the relative height of a stutter peak accurately so as to correct the experimental results of an individual typing experiment and a pooled typing experiment and to eliminate the need to perform an additional preliminary experiment, the following functions are required.

Function 1: DNA markers that are known to be compound markers and DNA markers that are known not to be compound markers are registered in a database in advance, and the database is referred to when estimating the relative height of a stutter peak. If a particular DNA marker is known to be not a compound marker, the relationship between the fragment length and the number of repetitions can be determined by referring to the sequence information, such as the human genome, without performing any additional preliminary experiment. On the contrary, even if the DNA marker is known to be a compound marker, the relationship between the fragment length and the number of repetitions can be known by comparatively observing the results of DNA sequencing experiments on many individuals, if such results are available. Thus, the first method for the calculation for estimating the relative height of a stutter peak from a fragment length can be utilized, without performing a DNA sequencing experiment as an additional preliminary experiment. This function is based on the aforementioned information 1.

Function 2-1: In a pooled typing experiment, by examining the intervals between peaks of a pooled typing waveform, it is determined whether or not a single-nucleotide in/del is included. This function can be realized by the procedure described with reference to the foregoing information 2.

Function 2-2: In an individual typing experiment, by examining the intervals between peaks of individual typing waveforms, it is determined whether or not a single-nucleotide in/del is included. This function can be realized by the procedure described with reference to the foregoing information 2.

Function 3: By examining, with reference to the published human genome sequence, whether or not a plurality of microsatellites with a number of repetitions are included, it is estimated whether a particular microsatellite is a compound marker. This function is based on the foregoing information 3.

Function 4: DNA markers that cannot be determined either to be compound markers or not by either function 1, function 2-1, function 2-2, or function 3 are estimated not to be compound markers. This function is based on the foregoing information 4.

With regard to DNA markers that cannot be determined either to be compound markers or not by any of the foregoing functions, the process can continue while presuming that such DNA markers are either compound markers or not. In an individual typing experiment, function 2-2 can be utilized, while in a pooled typing experiment, function 2-1 can be utilized. When a DNA marker is estimated not to be a compound marker by these functions, the relationship between the fragment length and the number of repetitions is estimated without performing an additional preliminary experiment. On the contrary, even when a DNA marker is estimated to be a compound marker, the following functions 5 and 6 can be utilized for many DNA markers.

Function 5: With regard to a compound marker that includes a single-nucleotide in/del and whose unit length is 3 nucleotides or longer, the relative height of a stutter peak is estimated by adjusting a linear regression line common to all of the DNA markers by a single nucleotide. This function is based on the foregoing information 5.

Function 6: With regard to DNA markers that include a plurality of microsatellites with polymorphisms in which unit lengths are the same, the relative height of a stutter peak is estimated by combining a plurality of linear regression lines. This function is based on the foregoing information 6.

Functions 5 and 6 allow the first method for determining the formula for estimating the relative height of a stutter peak based on the length of a fragment to be utilized for many of those DNA markers that are known to be compound markers but for which the experimental results of individual DNA sequencing cannot be utilized, or for many of those DNA markers that are estimated to be compound markers, without performing a DNA sequencing experiment as an additional preliminary experiment.

Function 7: Display function

The results of estimation of the relative height of a stutter peak using functions 1 to 6 are displayed on a screen. Thus, the user can be shown the results of estimation of the relative height of a stutter peak and the data on which the results are based.

In order to realize those functions mentioned above, the invention provides an apparatus for displaying the results of analysis of the length of a DNA fragment based on a detection signal obtained from a PCR amplification product of said DNA fragment, comprising:

a compound marker determination unit for determining whether or not said DNA fragment is a compound marker having a plurality of sequence portions with polymorphisms;
a relative height estimation unit for determining, based on the results of estimation made by said compound marker determination unit, whether or not it is possible to estimate the relative relationship between the height of a true peak that corresponds to said detection signal from said PCR amplification product of said DNA fragment and the height of a stutter peak that corresponds to a detection signal from said PCR amplification product in which the number of repetitions of a unit in a microsatellite in said DNA fragment has been increased or decreased; and
a display unit for displaying the results of determination made by said relative height estimation unit.

The apparatus further comprises a means for storing known information about compound markers, wherein said compound marker determination unit determines whether or not said DNA fragment is a compound marker using said known information, and wherein said relative height estimation unit determines whether or not a relative relationship between the height of a true peak and the height of a stutter peak can be estimated using said known information.

The invention further provides an apparatus for displaying the results of analysis of the length of a DNA fragment from a detection signal obtained from a PCR amplification product of said DNA fragment, comprising:

a compound marker determination unit for determining whether or not said DNA fragment is a compound marker having a plurality of sequence portions with polymorphism;
a relative height estimation unit for estimating, based on the results of estimation made by said compound marker determination unit, the relative relationship between the height of a true peak that corresponds to said detection signal from said PCR amplification product of said DNA fragment and the height of a stutter peak that corresponds to a detection signal from said PCR amplification product in which the number of repetitions of a unit in a microsatellite in said DNA fragment has been increased or decreased; and
a display unit for displaying the results of determination made by said relative height estimation unit.

The invention further provides an apparatus for displaying the results of analysis of the length of a DNA fragment from a detection signal obtained from a PCR amplification product of said DNA fragment, comprising:

a compound marker determination unit for determining whether or not said DNA fragment is a compound marker having a plurality of sequence portions with polymorphism;
a relative height estimation unit for determining, based on the results of estimation made by said compound marker determination unit, whether or not it is possible to estimate the relative relationship between the height of a true peak that corresponds to said detection signal from said PCR amplification product of said DNA fragment and the height of a stutter peak that corresponds to a detection signal from said PCR amplification product in which the number of repetitions of a unit in a microsatellite in said DNA fragment has been increased or decreased;
a correction unit for correcting said detection signal from said PCR amplification product of said DNA fragment based on the results of estimation made by said relative height estimation unit; and
a display unit for displaying the results of analysis of the length of said DNA fragment based on a corrected detection signal.

The apparatus further comprises a means for storing known information about compound markers, wherein said compound marker determination unit determining whether or not said DNA fragment is a compound marker using said known information, and wherein said relative height estimation unit determines whether or not a relative relationship between the height of a true peak and the height of a stutter peak can be estimated using said known information.

The compound marker determination unit determines whether or not, based on the intervals of peaks in a waveform of said detection signal of said PCR amplification product of said DNA fragment, said DNA fragment includes a single-nucleotide in/del.

The compound marker determination unit acquires information about the number of repetitions of a unit in a microsatellite included in said DNA fragment by referring to the published genome sequence of said DNA fragment.

The relative height estimation unit, when said DNA fragment includes a single-nucleotide in/del, adjusts the results of estimation based on a linear relationship between the length of said DNA fragment and the sum of the number of repetitions of a unit in each microsatellite included in said DNA fragment by referring to the published genome sequence of said DNA fragment.

The relative height estimation unit, when a plurality of microsatellites are included in said DNA fragment, adjusts the results of estimation based on a linear relationship between the length of said DNA fragment and the number of repetitions of a unit in each microsatellite included in said DNA fragment by referring to the published genome sequence of said DNA fragment.

The display unit displays information on which the estimation made by said relative height estimation unit is based.

The invention further provides a method for displaying the results of analysis of the length of a DNA fragment based on a detection signal obtained from a PCR amplification product of said DNA fragment, comprising the steps of:

determining whether or not said DNA fragment is a compound marker having a plurality of sequence portions with polymorphisms;
determining whether or not it is possible to estimate, based on the results of determination made in the compound marker determination step, a relative relationship between the height of a true peak that corresponds to said detection signal from said PCR amplification product of said DNA fragment and the height of a stutter peak that corresponds to a detection signal from a PCR amplification product in which the number of repetitions of a unit in a microsatellite of said DNA fragment has increased or decreased; and
displaying the results of determination made by the relative height estimation step.

The method further comprises the step of acquiring known information about compound markers prior to the compound marker determination step, wherein it is determined, using said known information, in the compound marker determination step whether or not said DNA fragment is a compound marker, and wherein it is determined in the relative height estimation step whether or not it is possible to estimate the relative relationship between the height of said true peak and the height of a stutter peak using said known information.

The invention further provides a method for displaying the results of analysis of the length of a DNA fragment based on a detection signal obtained from a PCR amplification product of said DNA fragment, comprising the steps of:

determining whether or not said DNA fragment is a compound marker having a plurality of sequence portions with polymorphisms;
estimating, based on the results of determination made by the compound marker determination step, the relative relationship between the height of a true peak that corresponds to said detection signal from said PCR amplification product of said DNA fragment and the height of a stutter peak that corresponds to a detection signal from a PCR amplification product in which the number of repetitions in a unit in a microsatellite of said DNA fragment has increased or decreased; and
displaying the results of estimation made by the relative height estimation step.

The invention further provides a method for displaying the results of analysis of the length of a DNA fragment based on a detection signal obtained from a PCR amplification product of said DNA fragment, comprising the steps of:

determining whether or not said DNA fragment is a compound marker having a plurality of sequence portions with polymorphism;
estimating, based on the results of determination made by the compound marker determination step, the relative relationship between the height of a true peak that corresponds to said detection signal from said PCR amplification product of said DNA fragment and the height of a stutter peak that corresponds to a detection signal from a PCR amplification product in which the number of repetitions in a unit in a microsatellite of said DNA fragment has increased or decreased;
correcting said detection signal from said PCR amplification product of said DNA fragment based on the results of estimation made by the relative height estimation step; and
displaying the results of analysis of the length of said DNA fragment based on a corrected detection signal.

The method further comprises the step of acquiring known information about compound markers prior to the compound marker determination step,
wherein it is determined, using said known information, in the compound marker determination step whether or not said DNA fragment is a compound marker,
and wherein it is determined in the relative height estimation step whether or not it is possible to estimate the relative relationship between the height of said true peak and the height of a stutter peak using said known information.

The compound marker determining step comprises determining, based on the intervals of peaks in the waveform of said detection signal of said PCR amplification product of said DNA fragment, whether or not said DNA fragment includes a single-nucleotide in/del.

The compound marker determination step comprises acquiring information about the number of repetitions of a unit in a microsatellite included in said DNA fragment by referring to the published genome sequence of said DNA fragment.

The relative height estimation step comprises adjusting, when a single-nucleotide in/del is included in said DNA fragment, the results of estimation by referring to the published genome sequence of said DNA fragment and estimation in accordance with a linear relationship between the length of said DNA fragment and the number of repetitions of a unit in a micro satellite included in said DNA fragment.

The relative height estimation step comprises adjusting, when a plurality of microsatellites are included in said DNA fragment, the results of estimation by referring to the published genome sequence of the DNA fragment and based on a linear relationship between the length of said DNA fragment and the sum of the number of repetitions of a unit in each microsatellite included in said DNA fragment.

The display step comprises displaying information on which the estimation made by the relative height estimation step is based.

The invention further provides a program for causing a computer to carry out any one of the foregoing methods.

As described above, in accordance with the method and apparatus for displaying gene information according to the invention, when the result of extracting and analyzing a DNA marker including a microsatellite with polymorphism or a single-nucleotide in/del by PCR and electrophoresis experiments, the experiment results that have already been obtained can be utilized for estimating the relative height of a stutter peak with high accuracy without conducting an additional preliminary experiment, whether in an individual typing experiment or a pooled typing experiment. The experimental results or the like can then be corrected by eliminating the influence of stutter peaks based on the estimation made.

In particular, in accordance with the method and apparatus for displaying gene information according to the invention, by applying a formula for estimating the relative height of a stutter peak based on the length of a fragment, the relative height of a stutter peak can be estimated, without requiring an additional experiment, for the following DNA markers that are yet to be known to be whether a compound marker or not: (1) DNA markers that include both a single-nucleotide in/del and a microsatellite with a unit length of 3 nucleotides or longer; (2) DNA markers that include a plurality of microsatellites with the same unit length; and (3) DNA markers that are actually not compound markers.

BRIEF DESCRIPTION OF THE DRAWINGS

Fig. 1 shows an example of a method for unequivocally calculating the number of repetitions of a microsatellite for a compound marker that includes a single-nucleotide in/del, based on the length of a PCR amplification product.

Fig. 2 schematically shows a functional block diagram of the internal structure of a gene information display system according to the invention.

Fig. 3 shows the data structure of marker data included in a data memory shown in Fig. 2.

Fig. 4 shows the data structure of pooled typing data included in the data memory shown in Fig. 2.

Fig. 5 shows the data structure of individual typing data included in the data memory shown in Fig. 2.

Fig. 6 schematically shows a flowchart of a process performed by the gene information display system shown in Fig. 2.

Fig. 7 shows a flowchart of the details of a process performed by a compound marker determination unit at step 601 of Fig. 6.

Fig. 8 shows a flowchart of the details of a process performed by a relative height estimation unit for estimating the relative height of a stutter peak at step 602 of Fig. 6.

Fig. 9 shows a display screen provided by the estimation result display unit at step 604 of Fig. 6.

Fig. 10 shows a detailed display screen displayed upon pressing of a detailed display button shown in Fig. 9.

Fig. 11 shows a display screen provided by the estimation result display unit at step 606 of Fig. 6.

Fig. 12 shows an example of a single-nucleotide in/del, which is a type of polymorphism in which individuals differ in terms of presence or absence of a single nucleotide at a single gene locus.

Fig. 13 shows examples of microsatellite that appears on the genome.

Fig. 14 schematically shows the procedure of an experiment in which a DNA fragment is extracted from a microsatellite portion and amplified by PCR and electrophoresis.

Fig. 15 schematically shows the procedure of a pooled typing experiment involving PCR and electrophoresis conducted on a group of samples collected from a plurality of individuals.

Fig. 16 shows DNA fragments that include two kinds of microsatellites with polymorphism as an example of a compound marker.

Fig. 17 shows DNA fragments that include a microsatellite with polymorphism and a single-nucleotide in/del as another example of a compound marker.

Fig. 18 schematically shows stutter peaks, which is a typical example of noise caused during the process of a PCR and electrophoresis experiment.

Fig. 19 illustrates a problem in a pooled typing experiment using a compound marker.

Fig. 20 illustrates another problem in a pooled typing experiment using a compound marker.

DESCRIPTION OF PREFERRED EMBODIMENTS

With reference to the attached drawings, preferred embodiments are described of the method and apparatus for displaying gene information utilizing a gene frequency estimation system based on the utilization of published genome sequences according to the invention. Figs. 1 to 11 show the embodiments of the invention, in which identical reference numerals designate identical elements with similar structures and operations.

Structure of a gene information display system

Fig. 2 shows a schematic functional block diagram of the internal structure of a gene information display system according to an embodiment of the invention. The gene information display system includes: a waveform database 200 in which waveform data obtained as a result of fluorescence analysis of a PCR amplification product following a PCR and electrophoresis experiment is stored; a genome sequence database 201 in which published information about the human genome sequence of DNA markers is stored; a display unit 202 for displaying waveform data, the published human genome sequence data, and the result of analyzing them in the from of graphs; a pointing device 204 including a keyboard 203 and a mouse for performing operations for selecting a nucleotide sequence, an individual, or a peak in the displayed data; a central processing unit (CPU) 205 for performing required computations and control processes; a program memory 206 in which programs necessary for the processes performed by the CPU 205 are stored; and a data memory 207 in which data required for the processes performed by the CPU 205 are stored.

The program memory 206 includes: a compound marker determination unit 208 for realizing the aforementioned functions 1, 2-1, 2-2, 3, and 4, namely, those functions for examining whether or not a DNA marker is a compound marker; a relative height estimation unit 209 for estimating the relative height of a stutter peak by realizing the aforementioned functions 1, 5, and 6, namely, functions for estimating the relative height of a stutter peak; an estimation result display unit 210 for displaying the results from the compound marker determination unit 208 and the relative height estimation unit 209; and an estimation result correction unit 211 for correcting waveform data using the results of estimation if the estimation is possible and a conventional technique.

The data memory 207 includes marker data 212 including the published human genome sequence data for each DNA marker, pooled typing data 213 including waveform data obtained as a result of a pooled typing experiment, and individual typing data 214 including waveform data obtained as a result of an individual typing experiment.

Fig. 3 shows the data structure of the marker data 212 included in the data memory 207. The data structure, or MarkerData [], includes, for a number i of DNA markers: marker name 300 for each DNA marker; genome sequence 301; an estimation impossibility flag 302 indicating whether or not the relative height of a stutter peak can be estimated without performing a preliminary experiment; a single-nucleotide in/del presence flag 303 indicating whether or not a single-nucleotide in/del is contained; a unit length list 304 in which the position of each microsatellite with polymorphism contained in a DNA marker and its unit length are listed in pairs; the relationship between the fragment length and the number of repetitions 305; and the relationship 306 between the fragment length and the relative height of a stutter peak.

The data 302 has a NULL value when no calculations have been made. The data 303 has a NULL value when no calculations have been made, or when it is unknown whether or not there is a single-nucleotide in/del. The data 304 has a NULL value when no calculations have been made, or when it is unknown whether or not the DNA marker is a compound marker that includes a plurality of microsatellite with polymorphism. The data 305 holds data in the form of a sequence of a data structure FragmentSizeRepeatNumberData, as will be described below, for DNA markers that are known to be compound markers and for which nucleotide sequence frequency information can be utilized. For other DNA markers, the data 305 has a NULL value.

The data structure FragmentSizeRepeatNumberData [] includes, for a number j of fragment lengths that a single DNA marker has as an allele, fragment lengths 307 and a list 308 of structures of microsatellites that correspond to the fragment lengths. The data 308 are stored in the form of sequences of a data structure RepeatNumberData. The data structure RepeatNumberData [] includes, regarding the structures of a number k of microsatellites that a single allele has, an intra-group proportion 309 and a microsatellite structure content 310. In the data shown in Fig. 3 by way of example, it can be seen that there are k alleles with the same fragment lengths that have different microsatellite structures, of which the first one has seven repetitions of AT and seven repetitions of ATG, and that the proportion of the alleles in a particular group that have this fragment length which have the particular microsatellite structure is 0.3.

Fig. 4 shows the data structure of the pooled typing data 213 included in the data memory 207. The data structure, or pooled typing data [], includes, for a single DNA marker for which a pooled typing experiment has been conducted, a marker name 400, waveform data 401 before correction obtained by the experiment, and corrected waveform data 402 obtained after correcting the waveform data 401 before correction using the results of estimation of the relative height of a stutter peak and a conventional technique. The data 401 and 402 are stored in the form of a list of the fragment length of each peak and fluorescent intensities. When the individual typing experiment has been conducted without performing a pooled typing experiment, the pooled typing data [] would be empty.

Fig. 5 shows the data structure of the individual typing data 214 included in the data memory 207. This data structure, or IndividualTypingData [], includes, for each of a number m of DNA markers for which individual typing experiments have been conducted, a marker name 500 and data 501 experimentally obtained for each individual. The data 501 is stored in the form of a sequence of data structure IndividualData[] as shown below. The data structure IndividualData[] includes, for a number n of individuals for which an individual typing experiment has been conducted with reference to a single DNA marker, an individual ID 502 for each individual, waveform data 503 obtained by the experiment, and a true peak 504 obtained using the results of estimation of the relative height of a stutter peak and a conventional technique. The data 503 is stored in the form of a list of pairs of the fragment length of each peak and fluorescent intensities. When the pooled typing experiment has been conducted without performing an individual typing experiment, the individual typing data [] would be empty.

<u>Process performed by the gene information display system</u>

In the following, a process performed by the gene information display system of the present embodiment, which is configured as described above, are described. Fig. 6 shows a flowchart of the process performed by the gene information display system.

With reference to Fig. 6, data regarding a DNA marker for which an experiment has been conducted is read from the waveform database 200 and the genome sequence database 201 (step 600). The data that has been read is then held in the data memory 207 in the form of marker data 212, pooled typing data 213, and individual typing data 214. When either a pooled typing experiment or an individual typing experiment has been conducted exclusively, either data 213 or data 214 is read. When it is known whether or not there is a single-nucleotide in/del in this DNA marker, TRUE or FALSE is stored in the single-nucleotide in/del presence flag 303 in the data structure MarkerData [] as shown in Fig. 3. When it is known whether or not the particular DNA marker is a compound marker that includes a plurality of microsatellites with polymorphism, a pair of the position of each microsatellite and its unit length is stored in the unit length list 304. When it is known that the particular DNA marker is a compound marker and when the nucleotide sequence frequency information is available, such information are stored in the relationship between the fragment length and the number of repetitions 305.

Thereafter, it is examined by the compound marker determination unit 208 whether or not the target DNA marker is a compound marker (step 601). This process will be described later with reference to Fig. 7. The result of the examination of whether or not the target DNA marker is a compound marker is stored in the single-nucleotide in/del flag presence 303 and the unit length list 304 of the data structure MarkerData [] shown in Fig. 3.

The relative height of a stutter peak that appears in the waveform data of the target DNA marker is then estimated by the relative height estimation unit 209 (step 602). This process will be later described in detail with reference to Fig. 8. When it is determined that the relative height of a stutter peak cannot be estimated, TRUE is set in the estimation impossibility flag 302 of the data structure MarkerData [] shown in Fig. 3. When the relative height of a stutter peak has been estimated, the results of estimation is stored in the relationship 306 between the fragment length and the relative height of a stutter peak of the data structure MarkerData []. Depending on whether or not TRUE has been set in the estimation impossibility flag, the subsequent process branches out in one way or the other (step 603).

When the estimation impossibility flag is not set to be TRUE, the results of estimation is displayed on the screen by the estimation result display unit 210 (step 604). The details of the display process will be described later with reference to Figs. 9 and 10. then, using the results of estimation obtained at step 602, the experimental result is corrected by the estimation result correction unit 211 (step 605). This experimental result correction process can be performed using a conventional technique and is therefore not described herein.

On the other hand, when the estimation impossibility flag is set to be TRUE, a message is displayed by the estimation result display unit 210 on the screen to the effect that an additional preliminary experiment is required (step 606). The process of this display will be described later in greater detail with reference to Fig. 11. Thereafter, the user enters the data obtained in the additional preliminary experiment (waveform data for the target DNA marker and data regarding the relative height of a stutter peak) into the system. The experimental result can then be corrected by the estimation result correction unit 211 using the data (step 607). Such experimental result correction process can be performed using a conventional technique and is therefore not described herein.

Process for examining whether or not a target DNA marker is a compound marker

With reference to a detailed flowchart shown in Fig. 7, the details are described of the process performed at step 601 of Fig. 6 for examining whether or not the target DNA marker is a compound marker. This flowchart consists of two major portions, namely, one in which it is examined whether or not the DNA marker is a compound marker with a single-nucleotide in/del, and the other, which comes later, in which it is examined whether or not the DNA marker is a compound marker that includes a plurality of microsatellites with polymorphism. First, it is examined whether or not it is known whether or not the target DNA marker is a compound marker that includes a single-nucleotide in/del (step 700). This condition would be satisfied if the single-nucleotide in/del presence flag 303 in the data structure MarkerData [] shown in Fig. 3 is not set to be a NULL value. When the condition is not met, the following process is performed. First, it is examined whether the data structure PooledTypingData [] shown in Fig. 4 is empty or not (i.e., whether a pooled typing experiment has been conducted or not), and whether peaks appear in this data structure at the intervals of a single nucleotide or (unit length - 1) nucleotides (step 701). If such peaks appear, it can be known using function 2-1 (information 2) that a single-nucleotide in/del is included, and, therefore, TRUE is stored in the single-nucleotide in/del presence flag 303 in the data structure MarkerData [] (step702). If the condition was not met at step 701, it is then examined whether or not the data structure IndividualTypingData [] shown in Fig. 5 is empty (i.e.; whether or not an individual typing experiment has been conducted), and whether or not peaks appear at the intervals of a single nucleotide or (unit length - 1) nucleotides in one or more pieces of individual data 501 of this data structure (step 703). If such peaks appear, it can be known using function 2-2 (information 2) that a single-nucleotide in/del is included, and, therefore, TRUE is stored in the single nucleotide in/del presence flag 303 in the data structure MarkerData [] (step 702). If the condition was not met at step 703, it can be known by functions 2-1 and 2-2 (information 2) that no single-nucleotide in/del is included, and, therefore, FALSE is stored in the single nucleotide in/del presence flag 303 (step 704). Thereafter, it is examined whether or not it is known whether or not a plurality of microsatellites with polymorphism are included (step 705). This condition would be met unless a NULL value is stored in the unit length list 304 in the data structure MarkerData []. If the condition is not met, the following process is performed. First, it is examined, with reference to the genome sequence 301 of the data structure MarkerData [], whether or not a plurality of microsatellites with a large number of repetitions are included (step 706). If a plurality of such microsatellites are included, a pair of the position of microsatellite and its unit length is stored in the unit length list 304 of the data structure MarkerData [] for each microsatellite (step 707). If there is only one microsatellite that is included at step 706, a pair of the position of that microsatellite and its unit length is stored in the unit length list 304 of the data structure MarkerData [] as a sole factor (step 708).

Process for determining the relationship between the fragment length and the relative height of a stutter peak

Details of the process for determining the relationship between the fragment length and the relative height of a stutter peak that is performed at step 602 shown in Fig. 6 will be described with reference to a detailed flowchart shown in Fig. 8. First, it is examined whether or not a target DNA marker is a compound marker and whether or not the relationship between the fragment length and the number of repetitions is available (step 800). This condition would be met unless the relationship between the fragment length and the number of repetitions 305 in the data structure MarkerData [] shown in Fig. 3 is set to be a NULL value. If the condition is met, the relationship between the fragment length and the relative height of a stutter peak is determined using the relationship between the fragment length and the number of repetitions and is stored in the relationship between the fragment length and the number of repetitions 306 of the data structure MarkerData [] (step 801). This determination can be made using a conventional technique on the basis of the aforementioned known property 3. then, FALSE is stored in the estimation impossibility flag 302 of the data structure MarkerData [] (step 802). If the condition was not met at step 800, the following process is carried out. First, it is examined whether or not a plurality of different unit lengths are registered in the unit length list 304 of the data structure MarkerData [] (step 803). This condition would not be met if, for example, two nucleotides have been registered twice as a unit length. If two nucleotides and three nucleotides have been registered once each as a unit length, the condition would be met. If the latter is the case, it would be impossible to estimate the relative height of a stutter peak without conducting an additional preliminary experiment and, therefore, TRUE is stored in the estimation impossibility flag 302 of the data structure MarkerData [] (step 804). If the condition was not met at step 803, the following process is carried out. First, it is examined whether or not the single nucleotide in/del presence flag 303 in the data structure MarkerData [] is TRUE (step 805). If TRUE, the following process is carried out. It is first examined whether or not the unit length registered in the unit length list 304 of the data structure MarkerData [] is less than 3 nucleotides (step 806). (Note that it is only necessary to take one nucleotide length as the unit length into consideration herein because of the branching process at step 803). If the unit length is less than 3 nucleotides, it is impossible to estimate the relative height of a stutter peak without an additional preliminary experiment, and, therefore, TRUE is stored in the estimation impossibility flag 302 of the data structure MarkerData [] (step 804). If the unit length was 3 nucleotides or longer at step 806, a linear regression line common to all of the DNA markers is adjusted by one nucleotide using function 5 (information 5) (step 807). If the single-nucleotide in/del presence flag 303 was FALSE at step 805, the following process is performed. First, it is examined whether a plurality of microsatellites with polymorphism are included (step 808). This condition would be met if a plurality of unit lengths are registered in the unit length list 304 of the data structure MarkerData []. If the condition is met, the relationship between the fragment length and the relative height of a stutter peak is determined by combining a plurality of linear regression lines using function 6 (information 6) and is then stored in the relationship 306 between the fragment length and the relative height of a stutter peak in the data structure MarkerData [] (step 809). Thereafter, FALSE is stored in the estimation impossibility flag 302 of the data structure MarkerData [] (step 810). If the condition was not met at step 808; a linear regression line common to all of the DNA markers is used as is using function 4 (information 4) so as to determine the relationship between the fragment length and the relative height of a stutter peak, which is then stored in the relationship 306 between the fragment length and the relative height of a stutter peak in the data structure MarkerData [] (step 811). Then, FALSE is stored in the estimation impossibility flag 302 of the data structure MarkerData [] (step 810).

With reference to an example of a display screen shown in Fig. 9, details of the screen display of the results of estimation of the relative height of a stutter peak that is performed at step 604 of Fig. 6 without performing an additional preliminary experiment. A graph is displayed with reference to the relationship 306 between the fragment length and the relative height of a stutter peak in the data structure MarkerData [] shown in Fig. 3 (900). The values shown on the horizontal axis of the graph indicate fragment lengths represented in terms of the number of nucleotides. The vertical axis shows the relative height of stutter peaks. Also, information about polymorphism included in a particular DNA marker is shown (901). What is displayed here is the outline of the information about polymorphism. By pressing a detailed display button 902, the information can be displayed in greater detail. Such a detailed display screen will be later described with reference to Fig. 10. What is displayed in 901 includes a display of the number of microsatellites with polymorphism in the form of a table according to unit lengths (903), and polymorphism other than microsatellites included in a particular DNA marker, namely, a display of the information about single-nucleotide in/del (904).

Fig. 10 shows an example of a detailed display screen that is shown by pressing the detailed display button 902 shown in Fig. 9. Information about microsatellites with polymorphism and single-nucleotide in/del included in a particular DNA marker is displayed with reference to the genome sequence 301 in the data structure MarkerData [], the unit length list 304, and the single nucleotide in/del presence flag 303 shown in Fig. 3 (1000). With regard to DNA markers that are known to be compound markers and for which nucleotide sequence frequency information is available, the structural contents of a microsatellite is displayed for each fragment length with reference to the relationship 305 between the fragment length and the number of repetitions of the data structure MarkerData [] (1001).

With reference to an example of a display screen shown in Fig. 11, details of a message displayed at step 606 of Fig. 6 indicating the necessity of an additional preliminary experiment is described. In a graph display 1100 of the relationship between the fragment length and the relative height of a stutter peak, a message is displayed noting the impossibility of estimating the relative height of a stutter peak without an additional preliminary experiment and a reason therefor (1101). Display of information about polymorphism included in a particular DNA marker (1102), display of a detailed display button (1103), display of the number of microsatellites with polymorphism in the form of a table according to unit lengths (1104), and display of information about polymorphism other than microsatellite included in a particular DNA marker, namely, information about single-nucleotide in/del (1105) are made in the same way as described with reference to Fig. 9. In addition to the information regarding the reason why the relative height of a stutter peak cannot be estimated, information indicating that it is unclear whether or not there is a single-nucleotide in/del is also useful for the user when making a decision as to what additional experiment is to be conducted. A detailed display screen that is shown by pressing the detailed display button 1103 is the same as that shown in Fig. 10.

While the invention has been described in the foregoing only with reference to a stutter peak as noise that is caused during the PCR and electrophoresis experiment processes, the invention can also be applied when a noise referred to as a +A peak is caused. This is due to the fact that functions 1, 3, 4, 5, 6, and 7, which do not involve waveform data, are not affected by +A peaks. Nor are functions 2-1 and 2-2 affected by +A peaks. As described in Patent Document 1, the way +A peaks appear in waveforms obtained in a single experiment conducted on a single sample (namely, the relative height of +A peaks relative to the original peaks) is substantially constant. Therefore, it can be concluded that, when no peaks appear at the unit length intervals, +A peaks appear and there is no single-nucleotide in/del if the ratio of height of two peaks that are spaced apart from one another by the length of a single nucleotide is constant, and that there is a single-nucleotide in/del if the ratio is not constant.

While the method and apparatus for displaying gene information according to the invention have been particularly shown and described with reference to preferred embodiments thereof, it will be understood by those skilled in the art that the foregoing and other changes in form and details can be made therein without departing from the spirit and scope of the invention.

While the human genome sequence information is currently open to the public, sequencing of the genomes of other animal species has not been completed and their sequence information that is available is limited. It goes without saying, however, that the method and apparatus for displaying gene information according to the invention will be able to utilize sequence information about other animal species when such sequence information is made public in the future.

The method and apparatus for displaying gene information can be realized on a computer having memory means, input means, display means, and so on. Information processing, such as the displaying of the result of a gene analysis experiment, estimation of a noise peak, and correction of experimental result based on the estimated result, can be performed using the aforementioned hardware resources including the memory means, input means, and display means. Thus, the invention can be industrially utilized.

## Claims

1. An apparatus for displaying the results of analysis of the length of a DNA fragment based on a detection signal obtained from a PCR amplification product of said DNA fragment, comprising:

   a compound marker determination unit for determining whether or not said DNA fragment is a compound marker having a plurality of sequence portions with polymorphism;
   a relative height estimation unit for determining, based on the results of determination made by said compound marker determination unit, whether or not it is possible to estimate the relative relationship between the height of a true peak that corresponds to said detection signal from said PCR amplification product of said DNA fragment and the height of a stutter peak that corresponds to a detection signal from said PCR amplification product in which the number of repetitions of a unit in a microsatellite in said DNA fragment has been increased or decreased; and
   a display unit for displaying the results of determination made by said relative height estimation unit.

2. The apparatus according to claim 1, further comprising a means for storing known information about compound markers,
   wherein said compound marker determination unit determining whether or not said DNA fragment is a compound marker using said known information, and wherein said relative height estimation unit determines whether or not a relative relationship between the height of a true peak and the height of a stutter peak can be estimated using said known information.

3. An apparatus for displaying the results of analysis of the length of a DNA fragment from a detection signal obtained from a PCR amplification product of said DNA fragment, comprising:

   a compound marker determination unit for determines whether or not said DNA fragment is a compound marker having a plurality of sequence portions with polymorphism;
   a relative height estimation unit for estimating, based on the results of determination made by said compound marker determination unit, the relative relationship between the height of a true peak that corresponds to said detection signal from said PCR amplification product of said DNA fragment and the height of a stutter peak that corresponds to a detection signal from said PCR amplification product in which the number of repetitions of a unit in a microsatellite in said DNA fragment has been increased or decreased; and
   a display unit for displaying the results of estimation made by said relative height estimation unit.

4. An apparatus for displaying the results of analysis of the length of a DNA fragment from a detection signal obtained from a PCR amplification product of said DNA fragment, comprising:

   a compound marker determination unit for determining whether or not said DNA fragment is a compound marker having a plurality of sequence portions with polymorphism;
   a relative height estimation unit for estimating, based on the results of determination made by said compound marker determination unit, the relative relationship between the height of a true peak that corresponds to said detection signal from said PCR amplification product of said DNA fragment and the height of a stutter peak that corresponds to a detection signal from said PCR amplification product in which the number of repetitions of a unit in a microsatellite in said DNA fragment has been increased or decreased;
   a correction unit for correcting said detection signal from said PCR amplification product of said DNA fragment based on the results of estimation made by said relative height estimation unit; and
   a display unit for displaying the results of analysis of the length of said DNA fragment based on a corrected detection signal.

5. The apparatus according to claim 3 or 4, further comprising a means for storing known information about compound markers,
   wherein said compound marker determination unit determining whether or not said DNA fragment is a compound marker using said known information, and wherein said relative height estimation unit determines the relative relationship between the height of a true peak and the height of a stutter peak using said known information.

6. The apparatus according to any one of claims 1 to 5, wherein said compound marker determination unit determines whether or not, based on the intervals of peaks in a waveform of said detection signal of said PCR amplification product of said DNA fragment, said DNA fragment includes a single-nucleotide in/del.

7. The apparatus according to any one of claims 1 to 6, wherein said compound marker determination unit acquires information about the number of repetitions of a unit in a microsatellite included in said DNA fragment by referring to the published genome sequence of said DNA fragment.

8. The apparatus according to any one of claims 3 to 7, wherein said relative height estimation unit, when said DNA fragment includes a single-nucleotide in/del, adjusts the results of estimation based on a linear relationship between the length of said DNA fragment and the number of repetitions of a unit in a microsatellite included in said DNA fragment by referring to the published genome sequence of said DNA fragment.

9. The apparatus according to any one of claims 3 to 8, wherein said relative height estimation unit, when a plurality of microsatellite are included in said DNA fragment, adjusts the results of estimation based on a linear relationship between the length of said DNA fragment and sum of the number of repetitions of a unit in each microsatellite included in said DNA fragment by referring to the published genome sequence of said DNA fragment.

10. The apparatus according to any one of claims 3 to 9, wherein said display unit displays information on which the estimation made by said relative height estimation unit is based.

11. A method for displaying the results of analysis of the length of a DNA fragment based on a detection signal obtained from a PCR amplification product of said DNA fragment, comprising:

a compound marker determination step for determining whether or not said DNA fragment is a compound marker having a plurality of sequence portions with polymorphism;
a relative height estimation step for determining, based on the results of determination made by said compound marker determination step, whether or not it is possible to estimate a relative relationship between the height of a true peak that corresponds to said detection signal from said PCR amplification product of said DNA fragment and the height of a stutter peak that corresponds to a detection signal from a PCR amplification product in which the number of repetitions in a unit in a microsatellite of said DNA fragment has increased or decreased; and
a display step for displaying the results of determination made by said relative height estimation step.

12. The method according to claim 11, further comprising the step of acquiring known information about compound markers prior to said compound marker determination step,
wherein it is determined, using said known information, in said compound marker determination step whether or not said DNA fragment is a compound marker,
and wherein it is determined in said relative height estimation step whether or not it is possible to estimate the relative relationship between the height of said true peak and the height of a stutter peak using said known information.

13. A method for displaying the results of analysis of the length of a DNA fragment based on a detection signal obtained from a PCR amplification product of said DNA fragment, comprising:

a compound marker determination step for determining whether or not said DNA fragment is a compound marker having a plurality of sequence portions with polymorphism;
a relative height estimation step for estimating, based on the results of determination made by said compound marker determination step, the relative relationship between the height of a true peak that corresponds to said detection signal from said PCR amplification product of said DNA fragment and the height of a stutter peak that corresponds to a detection signal from a PCR amplification product in which the number of repetitions of a unit in a microsatellite of said DNA fragment has increased or decreased; and
a display step for displaying the results of estimation made by said relative height estimation step.

14. A method for displaying the results of analysis of the length of a DNA fragment based on a detection signal obtained from a PCR amplification product of said DNA fragment, comprising:

a compound marker determination step for determining whether or not said DNA fragment is a compound marker having a plurality of sequence portions with polymorphism;
a relative height estimation step for estimating, based on the results of determination made by said compound marker determination step, the relative relationship between the height of a true peak that corresponds to said detection signal from said PCR amplification product of said DNA fragment and the height of a stutter peak that corresponds to a detection signal from a PCR amplification product in which the number of repetitions of a unit in a microsatellite of said DNA fragment has increased or decreased;

a correction step for correcting said detection signal from said PCR amplification product of said DNA fragment based on the results of estimation made by said relative height estimation step; and
a display step for displaying the results of analysis of the length of said DNA fragment based on a corrected detection signal.

**15.** The method according to claim 13 or 14, further comprising the step of acquiring known information about compound markers prior to said compound marker determination step,
wherein it is determined, using said known information, in said compound marker determination step whether or not said DNA fragment is a compound marker,
and wherein it is determined in said relative height estimation step whether or not it is possible to estimate the relative relationship between the height of said true peak and the height of a stutter peak using said known information.

**16.** The method according to any one of claims 11 to 15, wherein it is determined, based on the intervals of peaks in the waveform of said detection signal of said PCR amplification product of said DNA fragment, in said compound marker determination step whether or not said DNA fragment includes a single-nucleotide in/del.

**17.** The method according to any one of claims 11 to 16, wherein, in said compound marker determination step, information about the number of repetitions of a unit in a microsatellite included in said DNA fragment is acquired by referring to the published genome sequence of said DNA fragment.

**18.** The method according to any one of claims 13 to 17, wherein said relative height estimation step comprises adjusting, when a single-nucleotide in/del is included in said DNA fragment, the results of estimation by referring to the published genome sequence of said DNA fragment and in accordance with a linear relationship between the length of said DNA fragment and the number of repetitions of a unit in a microsatellite included in said DNA fragment.

**19.** The method according to any one of claims 13 to 18, wherein said relative height estimation step comprises referring to a published genome sequence of said DNA fragment and adjusting, when a plurality of microsatellites are included in said DNA fragment, the results of estimation based on a linear relationship between the length of said DNA fragment and sum of the number of repetitions of a unit in each microsatellite included in said DNA fragment.

**20.** The method according to any one of claims 13 to 19, wherein said display step comprises displaying information on which the estimation made by said relative height estimation step is based.

**21.** A program for causing a computer to carry out the method according to any one of claims 11 to 20.

FIG. 1

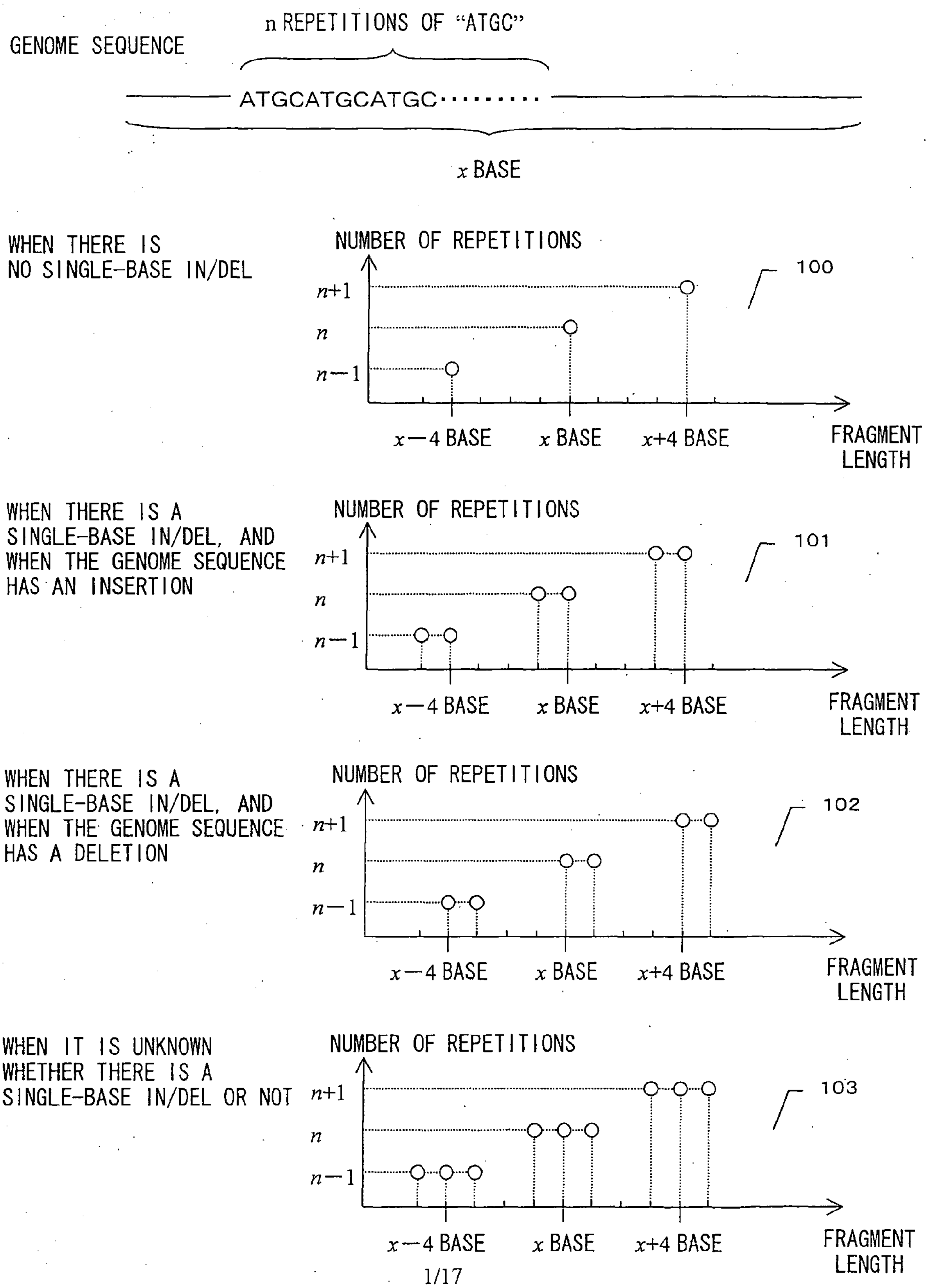
GENOME SEQUENCE
n REPETITIONS OF "ATGC"
ATGCATGCATGC·········
x BASE
WHEN THERE IS NO SINGLE-BASE IN/DEL
NUMBER OF REPETITIONS
n+1
n
n−1
x−4 BASE
x BASE
x+4 BASE
FRAGMENT LENGTH
100
WHEN THERE IS A SINGLE-BASE IN/DEL, AND WHEN THE GENOME SEQUENCE HAS AN INSERTION
NUMBER OF REPETITIONS
n+1
n
n−1
x−4 BASE
x BASE
x+4 BASE
FRAGMENT LENGTH
101
WHEN THERE IS A SINGLE-BASE IN/DEL, AND WHEN THE GENOME SEQUENCE HAS A DELETION
NUMBER OF REPETITIONS
n+1
n
n−1
x−4 BASE
x BASE
x+4 BASE
FRAGMENT LENGTH
102
WHEN IT IS UNKNOWN WHETHER THERE IS A SINGLE-BASE IN/DEL OR NOT
NUMBER OF REPETITIONS
n+1
n
n−1
x−4 BASE
x BASE
x+4 BASE
FRAGMENT LENGTH
103

1/17

FIG. 2

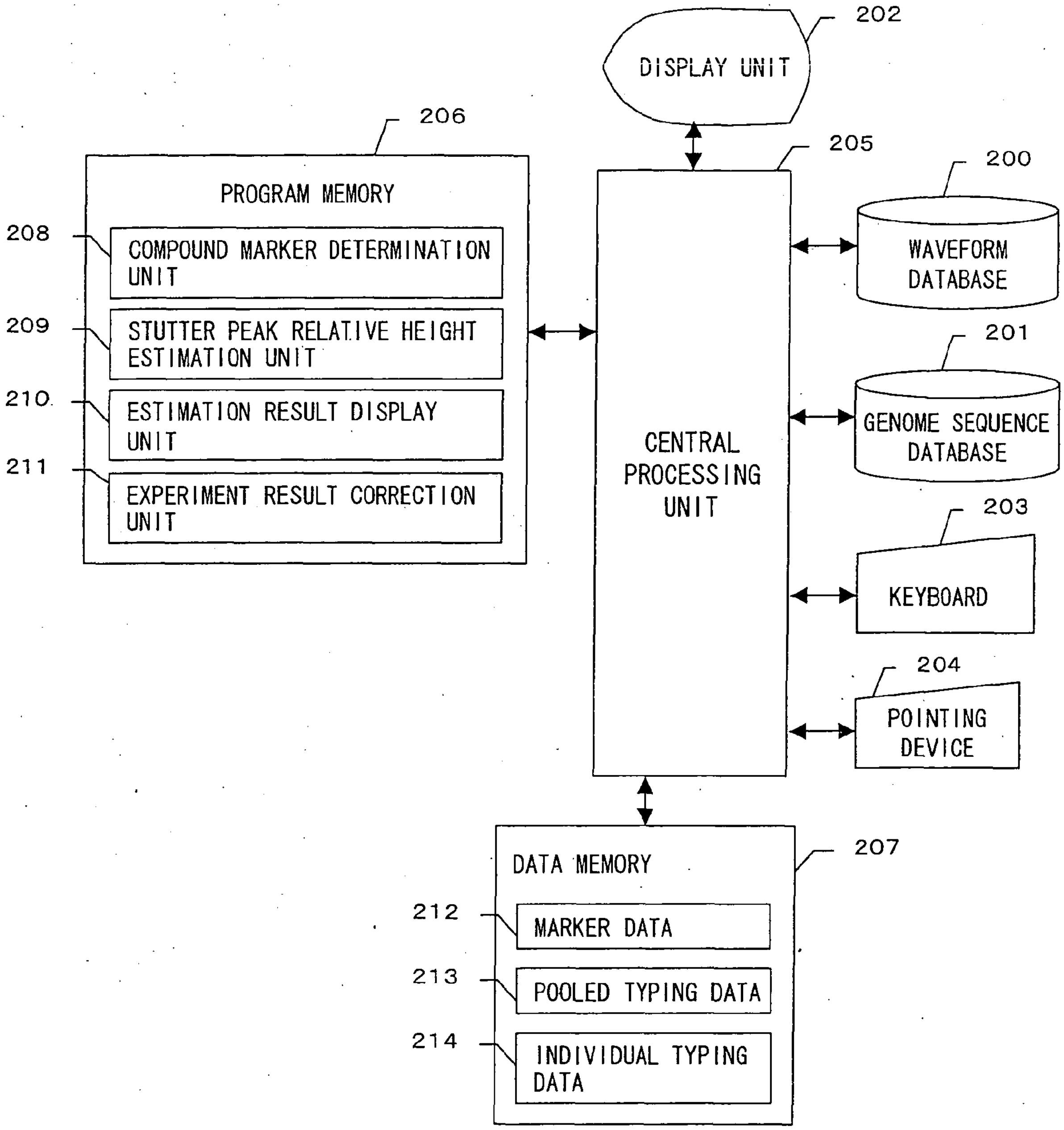

202
DISPLAY UNIT
206
205
200
PROGRAM MEMORY
208
COMPOUND MARKER DETERMINATION UNIT
WAVEFORM DATABASE
209
STUTTER PEAK RELATIVE HEIGHT ESTIMATION UNIT
201
210
ESTIMATION RESULT DISPLAY UNIT
GENOME SEQUENCE DATABASE
CENTRAL PROCESSING UNIT
211
EXPERIMENT RESULT CORRECTION UNIT
203
KEYBOARD
204
POINTING DEVICE
DATA MEMORY
207
212
MARKER DATA
213
POOLED TYPING DATA
214
INDIVIDUAL TYPING DATA

FIG. 3

MarkerData[ ]

| Ref. | MEMBER NAME | VALUE |
|---|---|---|
| 300 | MARKER NAME | Marker_00001 |
| 301 | GENOME SEQUENCE | "ATGCATGC..." |
| 302 | ESTIMATION IMPOSSIBILITY FLAG | NULL |
| 303 | SINGLE BASE IN/DEL PRESENCE FLAG | NULL |
| 304 | UNIT LENGTH LIST | [[34,2], [56,3]] |
| 305 | RELATION BETWEEN THE FRAGMENT LENGTH AND THE NUMBER OF REPETITIONS | FragmentSizeRepeatNumberData[] |
| 306 | RELATION BETWEEN THE FRAGMENT LENGTH AND THE RELATIVE HEIGHT OF A STUTTER PEAK | [[58, 0.4], [60, 0.42], ...] |

[1 ] [2 ] ... [i ]

FragmentSizeRepeatNumberData[ ]

| Ref. | MEMBER NAME | VALUE |
|---|---|---|
| 307 | FRAGMENT LENGTH | 58 |
| 308 | LIST OF MICROSATELLITE STRUCTURE | RepeatNumberData[ ] |

[1 ] [2 ] ... [j ]

RepeatNumberData[ ]

| Ref. | MEMBER NAME | VALUE |
|---|---|---|
| 309 | INTRA-GROUP RATIO | 0.3 |
| 310 | MICROSATELLITE STRUCTURE | [[AT, 7], [ATG, 7]] |
| | MICROSATELLITE STRUCTURE | [[AT, 4], [ATG, 9]] |
| | MICROSATELLITE STRUCTURE | [[AT, 10], [ATG, 5]] |
| | MICROSATELLITE STRUCTURE | [[AT, 13], [ATG, 3]] |

[1 ] [2 ] ... [k ]

FIG. 4

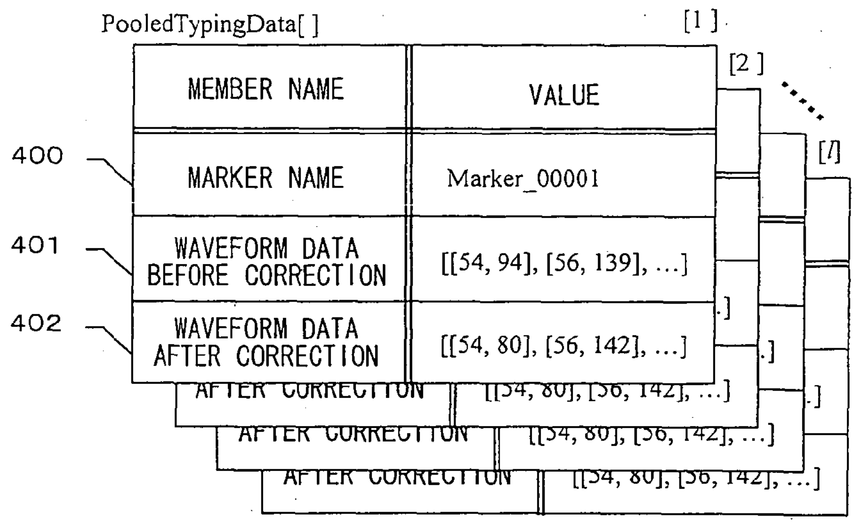
PooledTypingData[ ]
[1 ]
[2 ]
[/]
MEMBER NAME
VALUE
400
MARKER NAME
Marker_00001
401
WAVEFORM DATA BEFORE CORRECTION
[[54, 94], [56, 139], ...]
402
WAVEFORM DATA AFTER CORRECTION
[[54, 80], [56, 142], ...]

FIG. 5

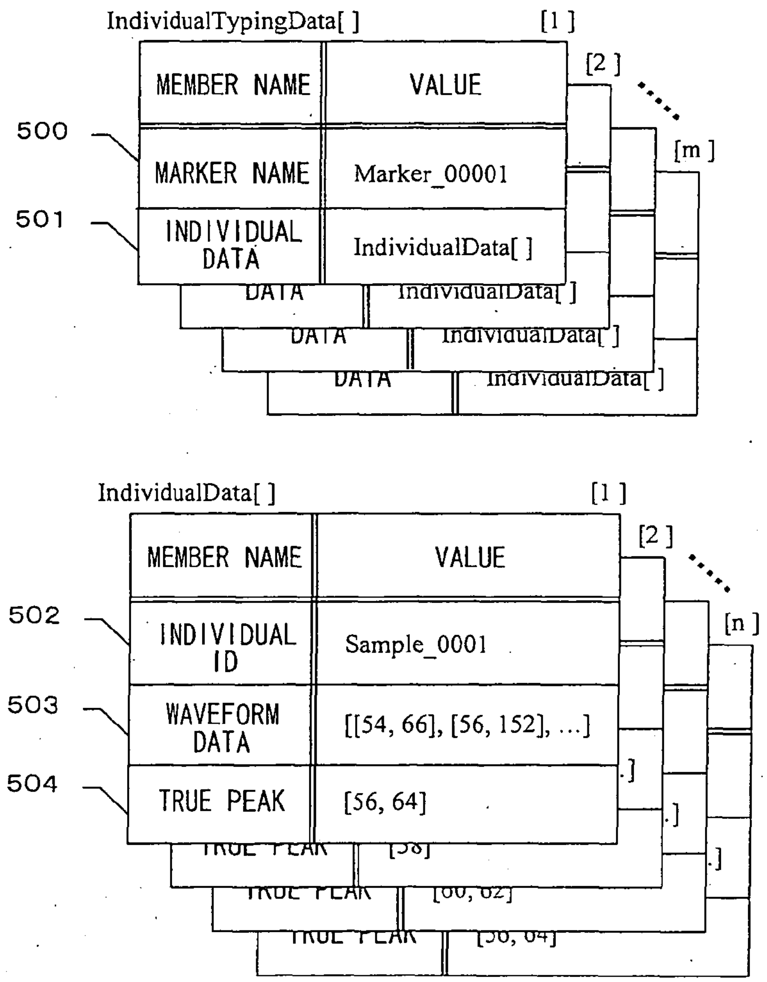
IndividualTypingData[ ]
[1 ]
[2 ]
[m ]
MEMBER NAME
VALUE
500
MARKER NAME
Marker_00001
501
INDIVIDUAL DATA
IndividualData[ ]
IndividualData[ ]
[1 ]
[2 ]
[n ]
MEMBER NAME
VALUE
502
INDIVIDUAL ID
Sample_0001
503
WAVEFORM DATA
[[54, 66], [56, 152], ...]
504
TRUE PEAK
[56, 64]

FIG. 6

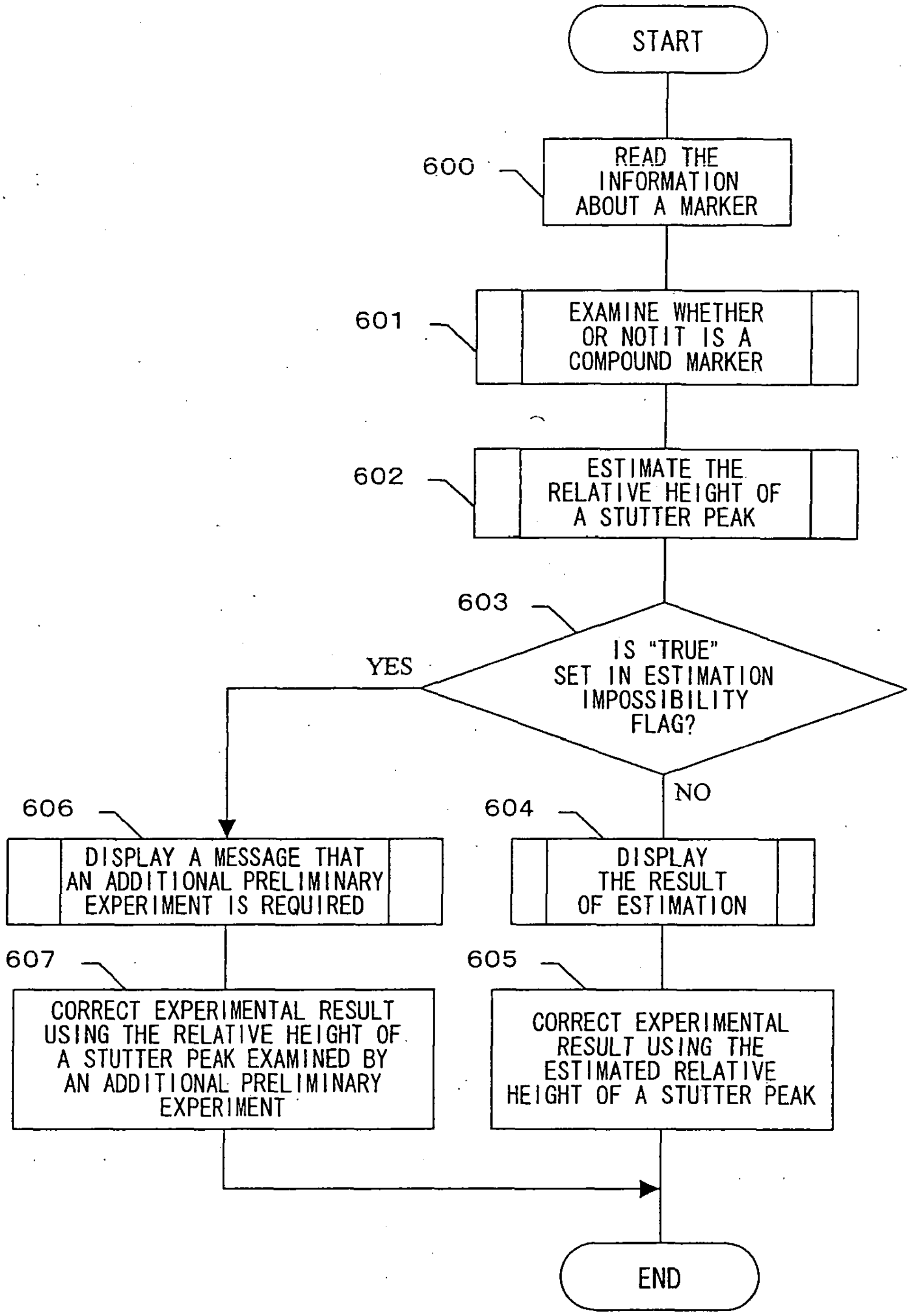
START
600
READ THE INFORMATION ABOUT A MARKER
601
EXAMINE WHETHER OR NOTIT IS A COMPOUND MARKER
602
ESTIMATE THE RELATIVE HEIGHT OF A STUTTER PEAK
603
IS "TRUE" SET IN ESTIMATION IMPOSSIBILITY FLAG?
YES
NO
606
DISPLAY A MESSAGE THAT AN ADDITIONAL PRELIMINARY EXPERIMENT IS REQUIRED
604
DISPLAY THE RESULT OF ESTIMATION
607
CORRECT EXPERIMENTAL RESULT USING THE RELATIVE HEIGHT OF A STUTTER PEAK EXAMINED BY AN ADDITIONAL PRELIMINARY EXPERIMENT
605
CORRECT EXPERIMENTAL RESULT USING THE ESTIMATED RELATIVE HEIGHT OF A STUTTER PEAK
END

FIG. 7

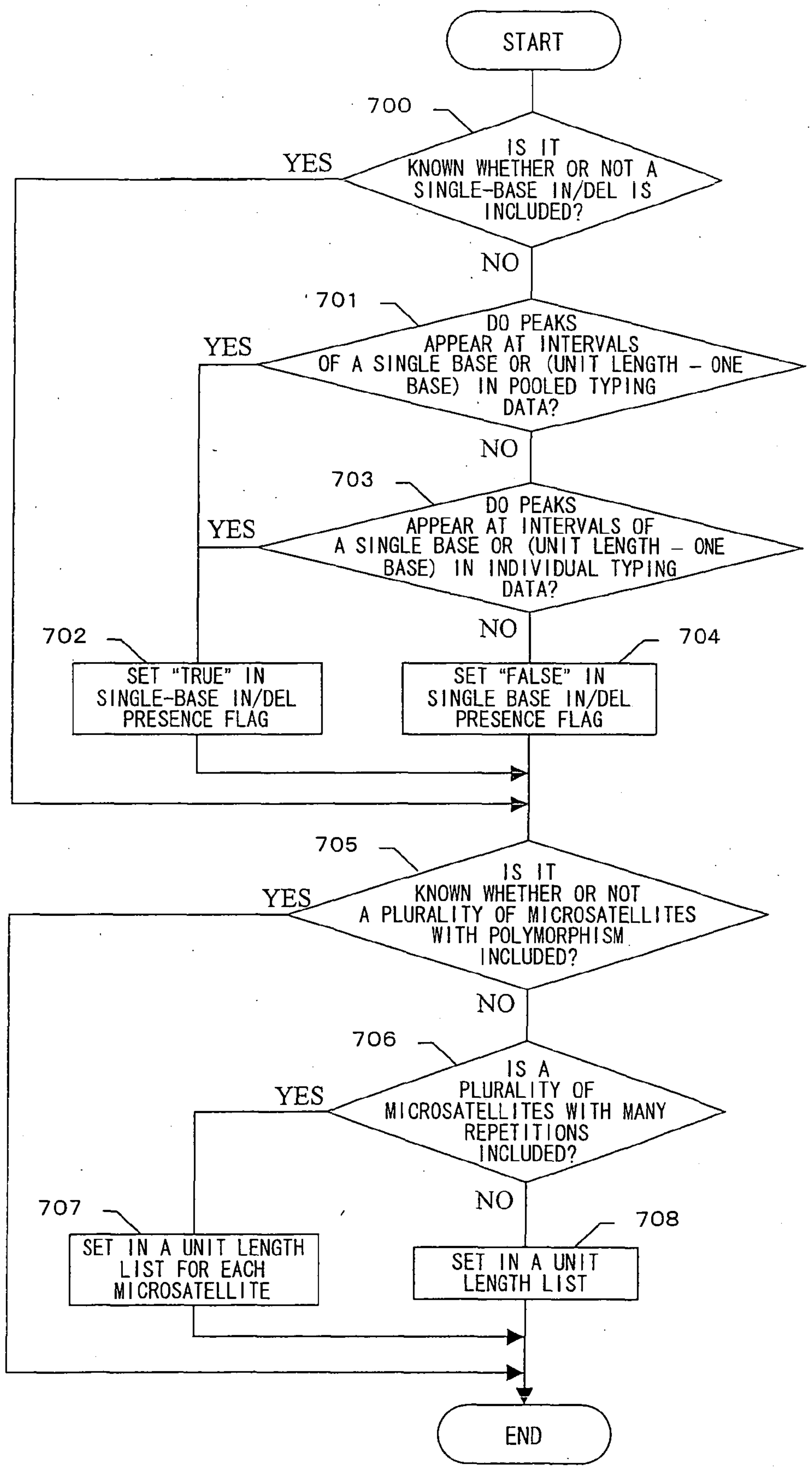
START
700
IS IT KNOWN WHETHER OR NOT A SINGLE-BASE IN/DEL IS INCLUDED?
YES
NO
701
DO PEAKS APPEAR AT INTERVALS OF A SINGLE BASE OR (UNIT LENGTH - ONE BASE) IN POOLED TYPING DATA?
YES
NO
703
DO PEAKS APPEAR AT INTERVALS OF A SINGLE BASE OR (UNIT LENGTH - ONE BASE) IN INDIVIDUAL TYPING DATA?
YES
NO
702
SET "TRUE" IN SINGLE-BASE IN/DEL PRESENCE FLAG
704
SET "FALSE" IN SINGLE BASE IN/DEL PRESENCE FLAG
705
IS IT KNOWN WHETHER OR NOT A PLURALITY OF MICROSATELLITES WITH POLYMORPHISM INCLUDED?
YES
NO
706
IS A PLURALITY OF MICROSATELLITES WITH MANY REPETITIONS INCLUDED?
YES
NO
707
SET IN A UNIT LENGTH LIST FOR EACH MICROSATELLITE
708
SET IN A UNIT LENGTH LIST
END

FIG. 8

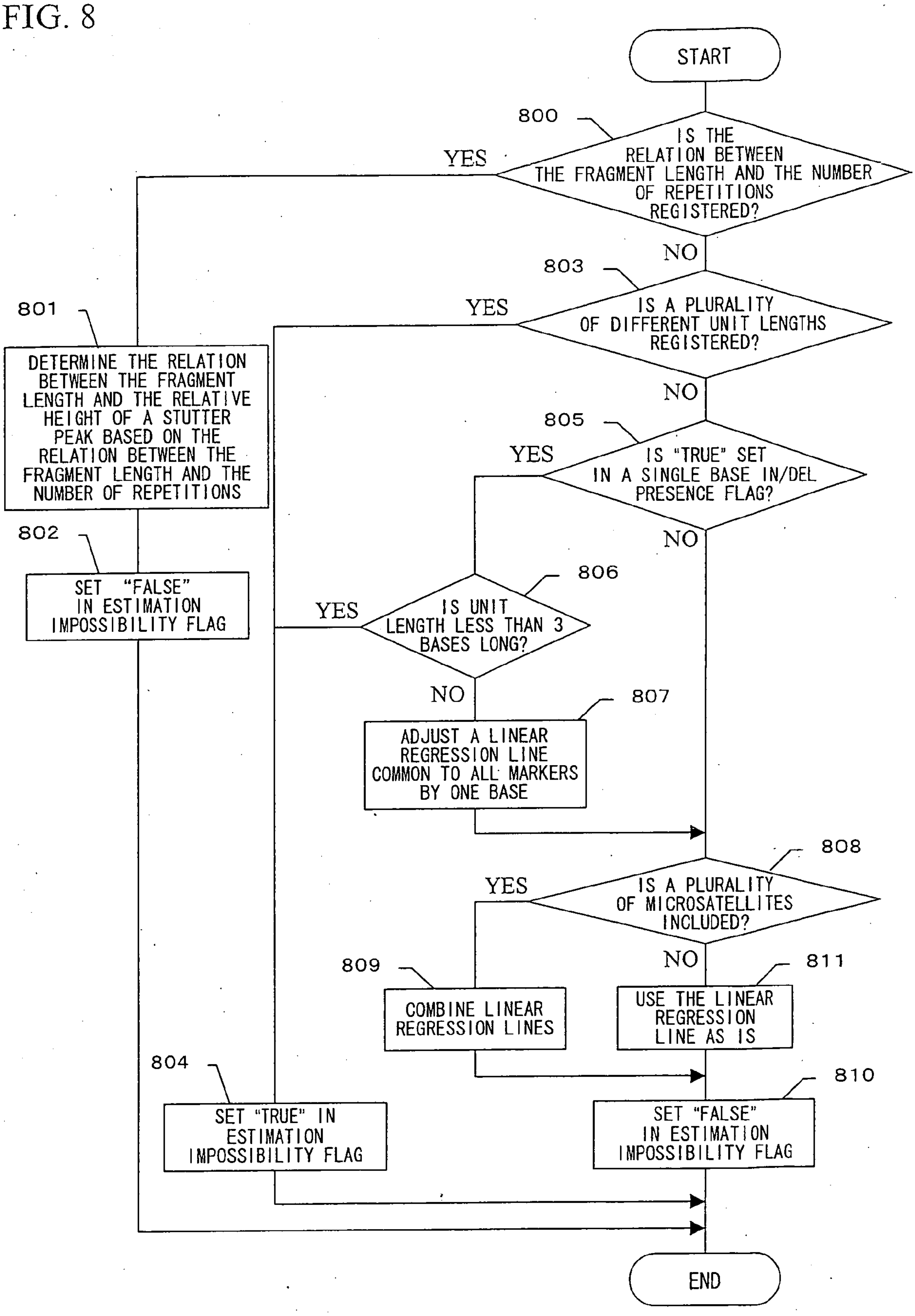
START
800
IS THE RELATION BETWEEN THE FRAGMENT LENGTH AND THE NUMBER OF REPETITIONS REGISTERED?
YES
NO
803
IS A PLURALITY OF DIFFERENT UNIT LENGTHS REGISTERED?
YES
NO
801
DETERMINE THE RELATION BETWEEN THE FRAGMENT LENGTH AND THE RELATIVE HEIGHT OF A STUTTER PEAK BASED ON THE RELATION BETWEEN THE FRAGMENT LENGTH AND THE NUMBER OF REPETITIONS
805
IS "TRUE" SET IN A SINGLE BASE IN/DEL PRESENCE FLAG?
YES
NO
802
SET "FALSE" IN ESTIMATION IMPOSSIBILITY FLAG
806
IS UNIT LENGTH LESS THAN 3 BASES LONG?
YES
NO
807
ADJUST A LINEAR REGRESSION LINE COMMON TO ALL MARKERS BY ONE BASE
808
IS A PLURALITY OF MICROSATELLITES INCLUDED?
YES
NO
809
COMBINE LINEAR REGRESSION LINES
811
USE THE LINEAR REGRESSION LINE AS IS
804
SET "TRUE" IN ESTIMATION IMPOSSIBILITY FLAG
810
SET "FALSE" IN ESTIMATION IMPOSSIBILITY FLAG
END

FIG. 9

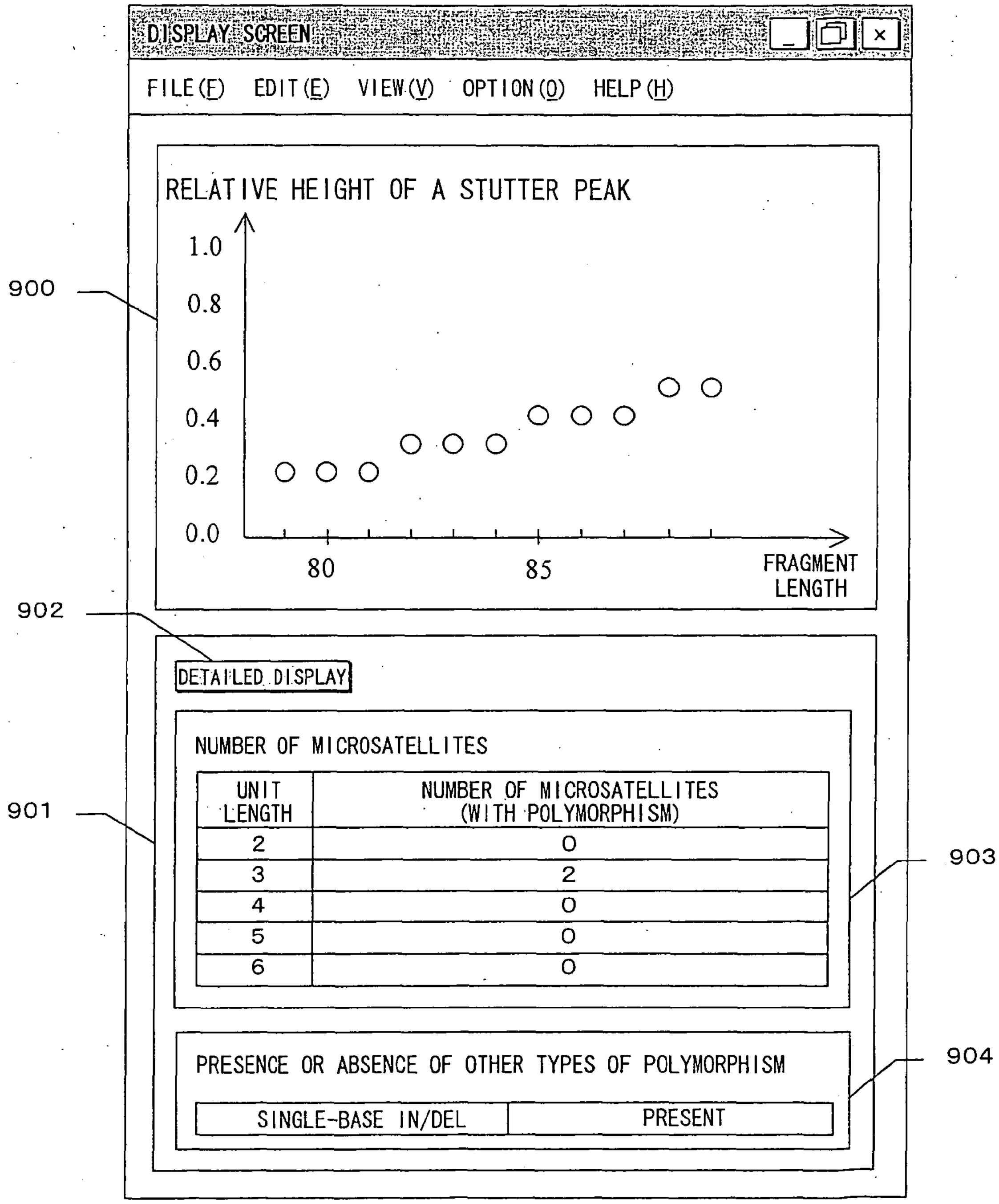
DISPLAY SCREEN
FILE(F) EDIT(E) VIEW(V) OPTION(O) HELP(H)
RELATIVE HEIGHT OF A STUTTER PEAK
1.0
0.8
0.6
0.4
0.2
0.0
80
85
FRAGMENT LENGTH
DETAILED DISPLAY
NUMBER OF MICROSATELLITES
UNIT LENGTH
NUMBER OF MICROSATELLITES (WITH POLYMORPHISM)
2
0
3
2
4
0
5
0
6
0
PRESENCE OR ABSENCE OF OTHER TYPES OF POLYMORPHISM
SINGLE-BASE IN/DEL
PRESENT
900
901
902
903
904

FIG. 10

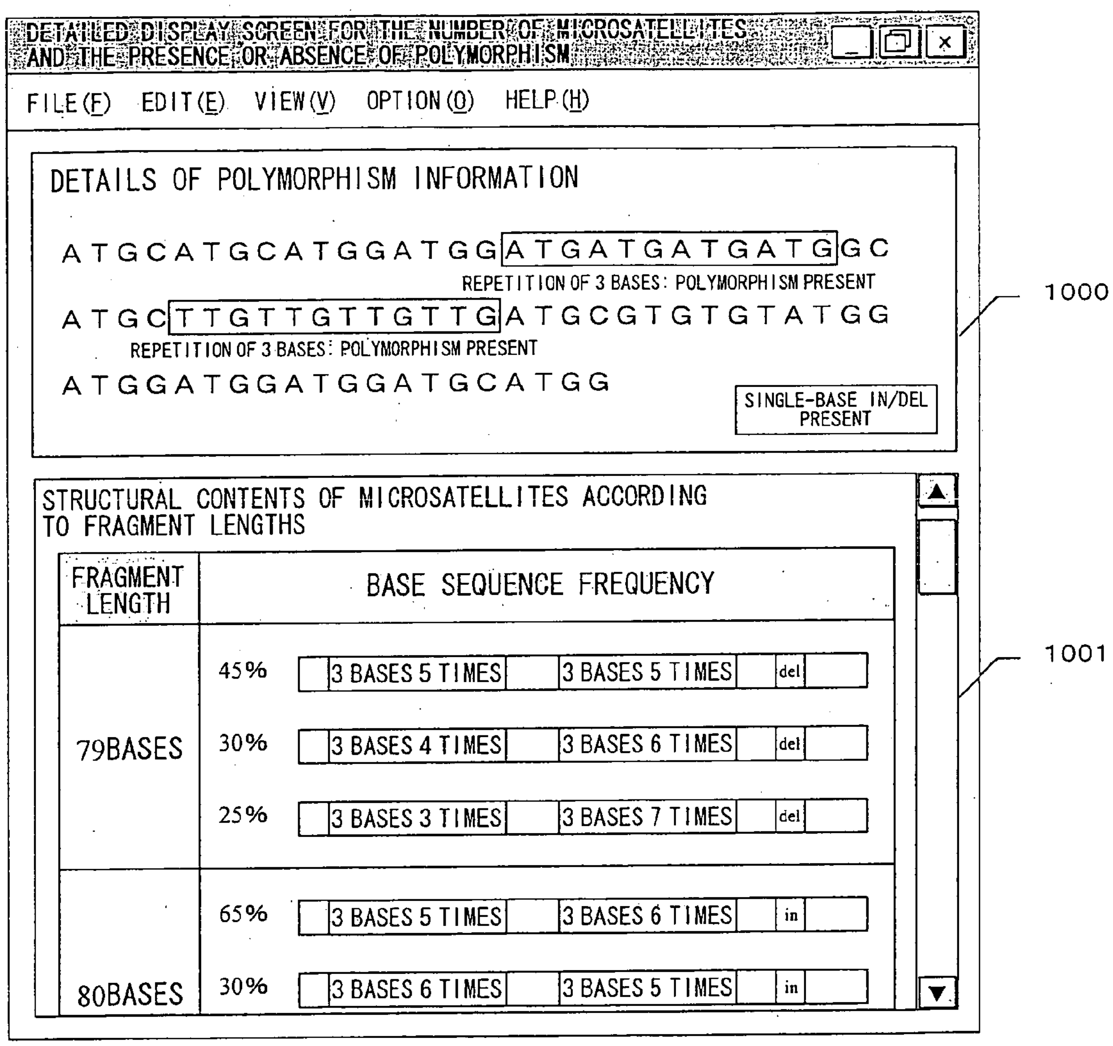
DETAILED DISPLAY SCREEN FOR THE NUMBER OF MICROSATELLITES AND THE PRESENCE OR ABSENCE OF POLYMORPHISM
FILE(F) EDIT(E) VIEW(V) OPTION(O) HELP(H)
DETAILS OF POLYMORPHISM INFORMATION
ATGCATGCATGGATGGATGATGATGATGGC
REPETITION OF 3 BASES: POLYMORPHISM PRESENT
ATGCTTGTTGTTGTTGATGCGTGTGTATGG
REPETITION OF 3 BASES: POLYMORPHISM PRESENT
ATGGATGGATGGATGCATGG
SINGLE-BASE IN/DEL PRESENT
1000
STRUCTURAL CONTENTS OF MICROSATELLITES ACCORDING TO FRAGMENT LENGTHS
FRAGMENT LENGTH
BASE SEQUENCE FREQUENCY
79BASES
45% 3 BASES 5 TIMES 3 BASES 5 TIMES del
30% 3 BASES 4 TIMES 3 BASES 6 TIMES del
25% 3 BASES 3 TIMES 3 BASES 7 TIMES del
80BASES
65% 3 BASES 5 TIMES 3 BASES 6 TIMES in
30% 3 BASES 6 TIMES 3 BASES 5 TIMES in
1001

FIG. 11

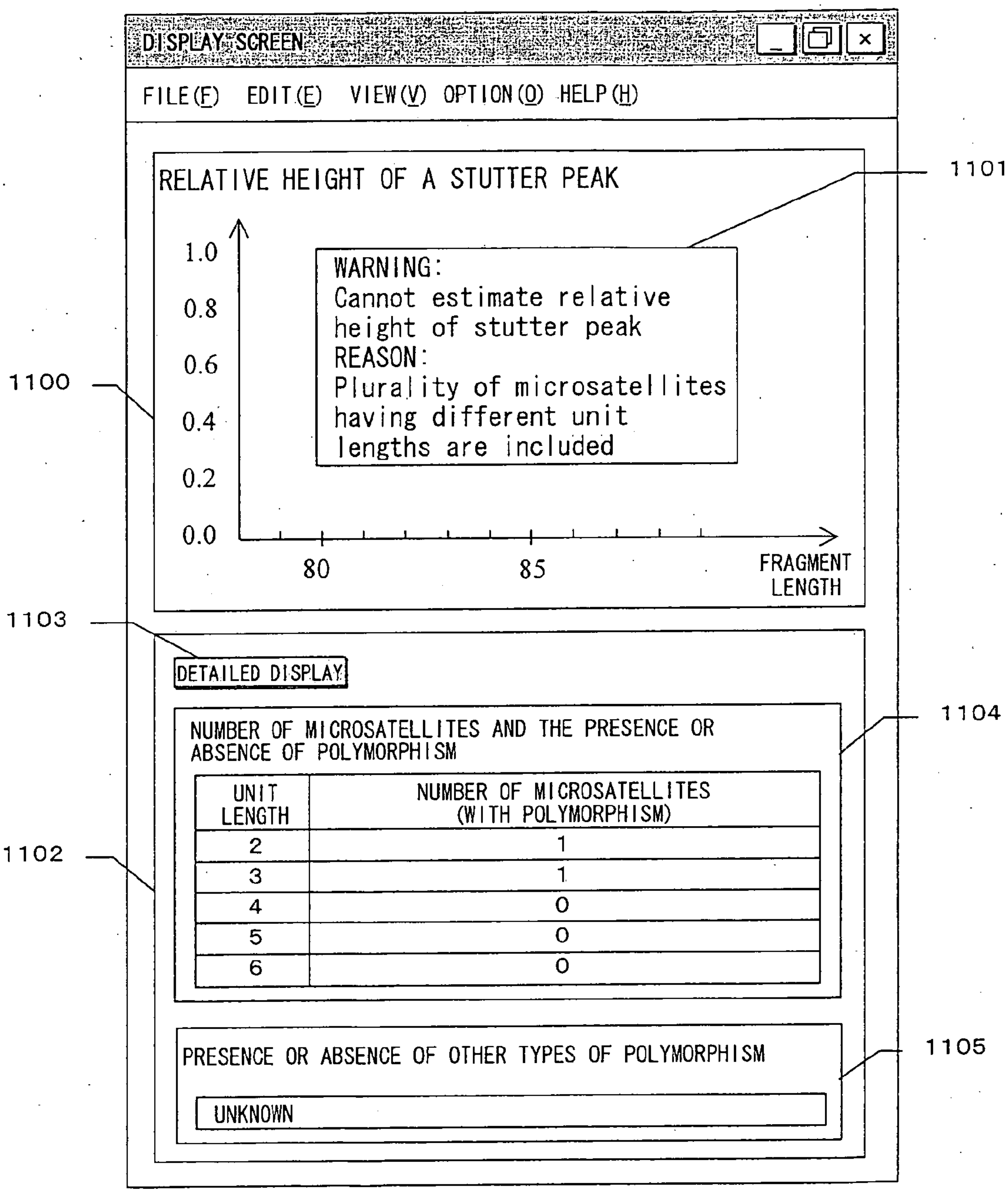
DISPLAY SCREEN
FILE(F) EDIT(E) VIEW(V) OPTION(O) HELP(H)
RELATIVE HEIGHT OF A STUTTER PEAK
1.0
0.8
0.6
0.4
0.2
0.0
80
85
FRAGMENT LENGTH
WARNING:
Cannot estimate relative height of stutter peak
REASON:
Plurality of microsatellites having different unit lengths are included
DETAILED DISPLAY
NUMBER OF MICROSATELLITES AND THE PRESENCE OR ABSENCE OF POLYMORPHISM
UNIT LENGTH
NUMBER OF MICROSATELLITES (WITH POLYMORPHISM)
2
1
3
1
4
0
5
0
6
0
PRESENCE OR ABSENCE OF OTHER TYPES OF POLYMORPHISM
UNKNOWN
1100
1101
1102
1103
1104
1105

FIG. 12

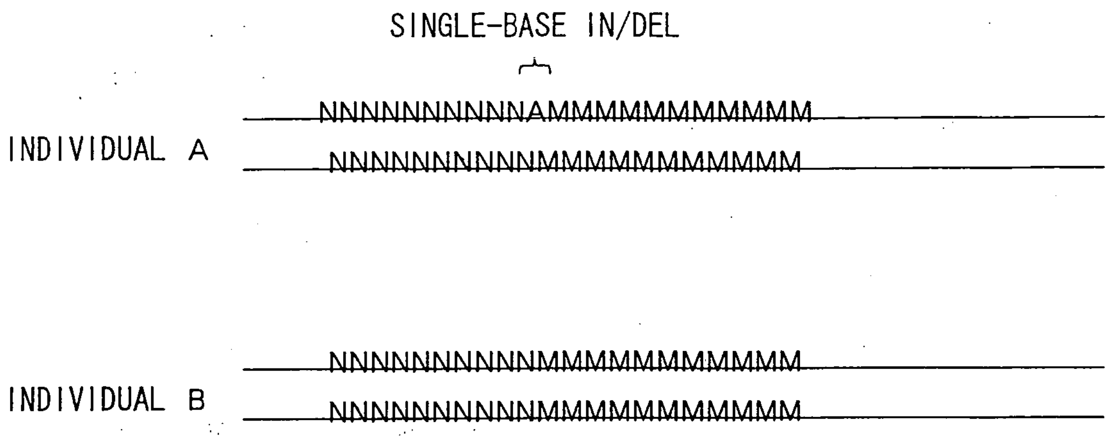
SINGLE-BASE IN/DEL
INDIVIDUAL A
NNNNNNNNNNAMMMMMMMMMMMM
NNNNNNNNNNMMMMMMMMMMMM
INDIVIDUAL B
NNNNNNNNNNMMMMMMMMMMMM
NNNNNNNNNNMMMMMMMMMMMM

FIG. 13

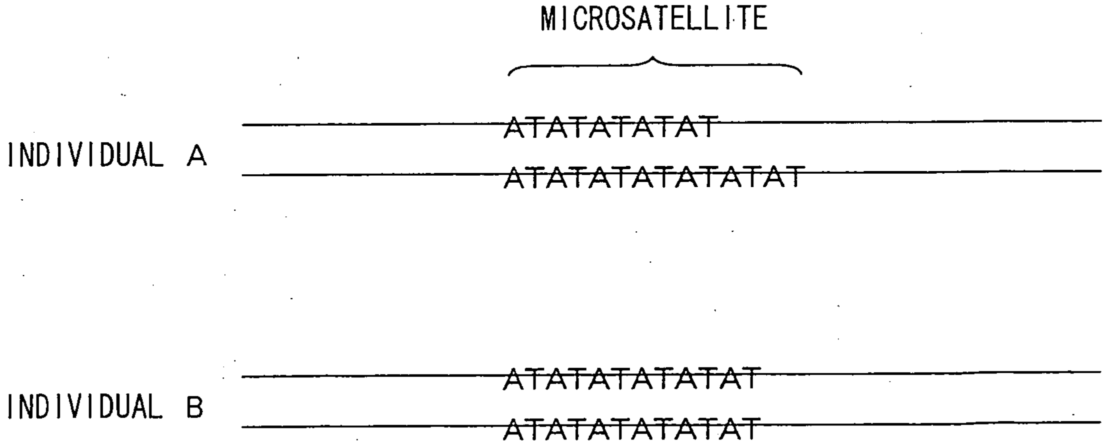
MICROSATELLITE
INDIVIDUAL A
ATATATATAT
ATATATATATATAT
INDIVIDUAL B
ATATATATATAT
ATATATATATAT

FIG. 14

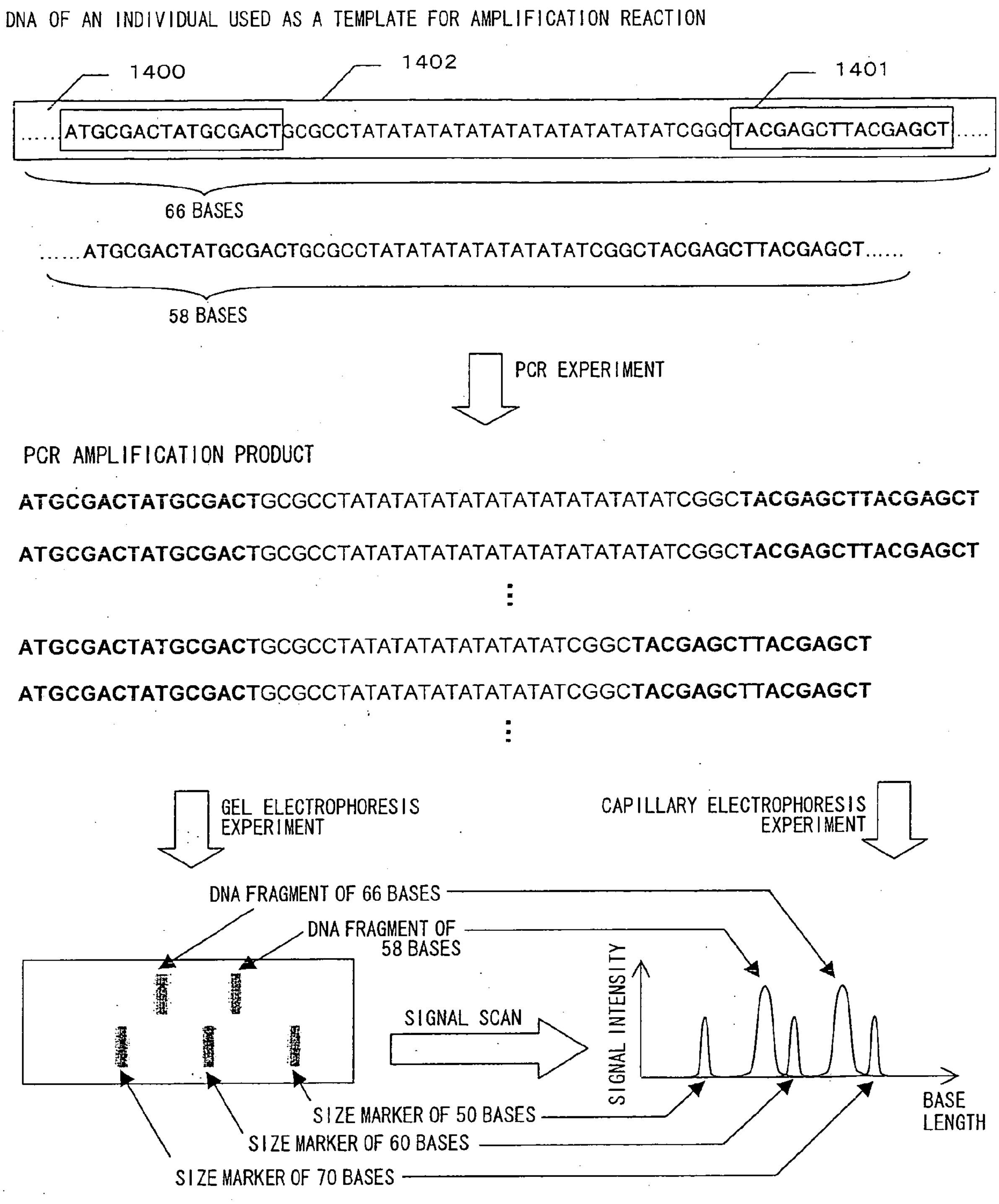
DNA OF AN INDIVIDUAL USED AS A TEMPLATE FOR AMPLIFICATION REACTION
1400
1402
1401
.....ATGCGACTATGCGACTGCGCCTATATATATATATATATATATATATCGGCTACGAGCTTACGAGCT.....
66 BASES
......ATGCGACTATGCGACTGCGCCTATATATATATATATATCGGCTACGAGCTTACGAGCT......
58 BASES
PCR EXPERIMENT
PCR AMPLIFICATION PRODUCT
ATGCGACTATGCGACTGCGCCTATATATATATATATATATATATATATCGGCTACGAGCTTACGAGCT
ATGCGACTATGCGACTGCGCCTATATATATATATATATATATATATATCGGCTACGAGCTTACGAGCT
⋮
ATGCGACTATGCGACTGCGCCTATATATATATATATATCGGCTACGAGCTTACGAGCT
ATGCGACTATGCGACTGCGCCTATATATATATATATATCGGCTACGAGCTTACGAGCT
⋮
GEL ELECTROPHORESIS EXPERIMENT
CAPILLARY ELECTROPHORESIS EXPERIMENT
DNA FRAGMENT OF 66 BASES
DNA FRAGMENT OF 58 BASES
SIGNAL SCAN
SIGNAL INTENSITY
BASE LENGTH
SIZE MARKER OF 50 BASES
SIZE MARKER OF 60 BASES
SIZE MARKER OF 70 BASES

FIG. 15

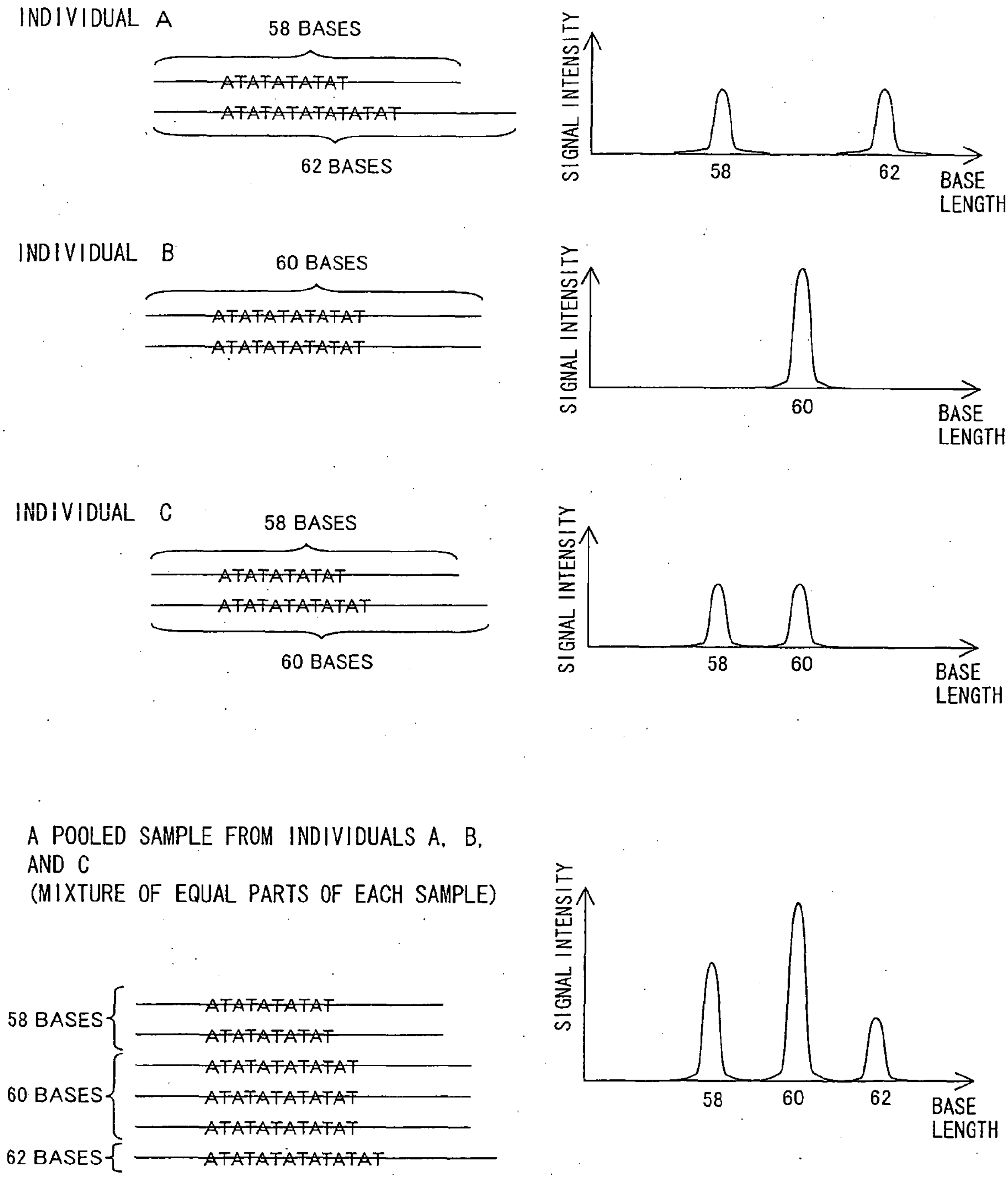


| ALLELE | 58 | 60 | 62 |
|---|---|---|---|
| PERCENTAGE | 33.2% | 50.0% | 16.7% |
| FREQUENCY | 2 | 3 | 1 |

FIG. 16

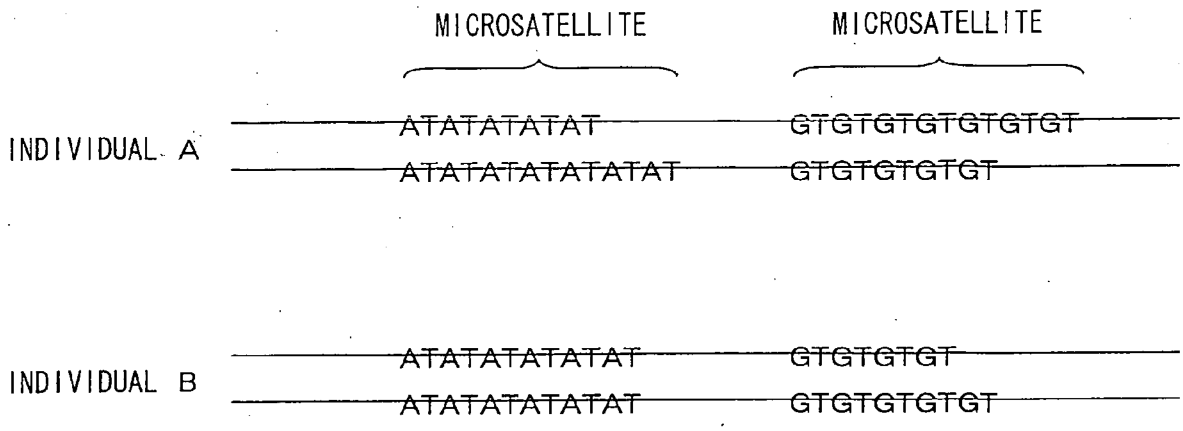
MICROSATELLITE
MICROSATELLITE
INDIVIDUAL A
ATATATATAT
GTGTGTGTGTGTGT
ATATATATATATAT
GTGTGTGTGT
INDIVIDUAL B
ATATATATATAT
GTGTGTGT
ATATATATATAT
GTGTGTGTGT

FIG. 17

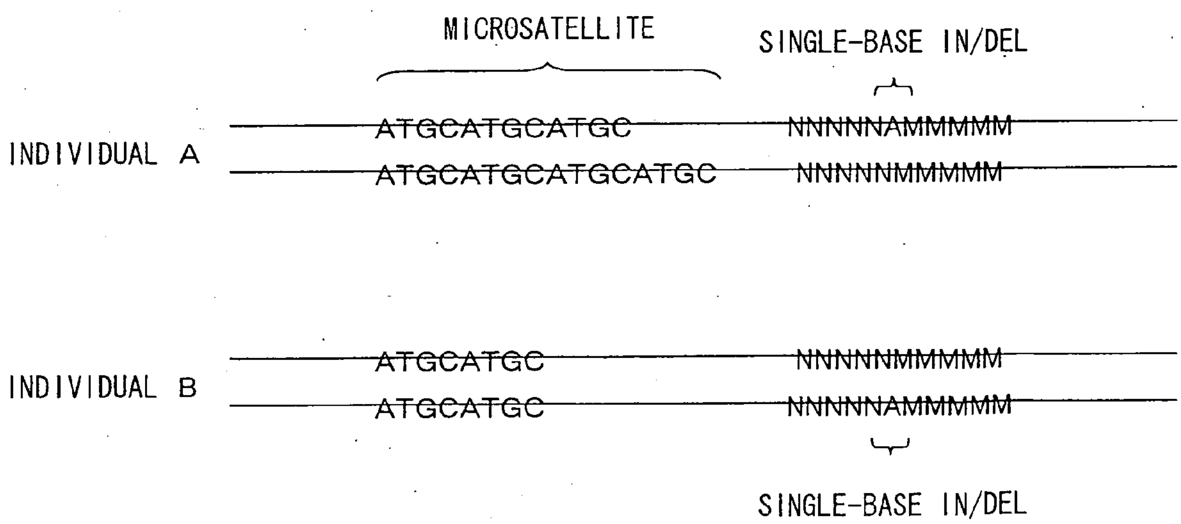
MICROSATELLITE
SINGLE-BASE IN/DEL
INDIVIDUAL A
ATGCATGCATGC
NNNNNAMMMMM
ATGCATGCATGCATGC
NNNNNMMMMM
INDIVIDUAL B
ATGCATGC
NNNNNMMMMM
ATGCATGC
NNNNNAMMMMM
SINGLE-BASE IN/DEL

FIG. 18

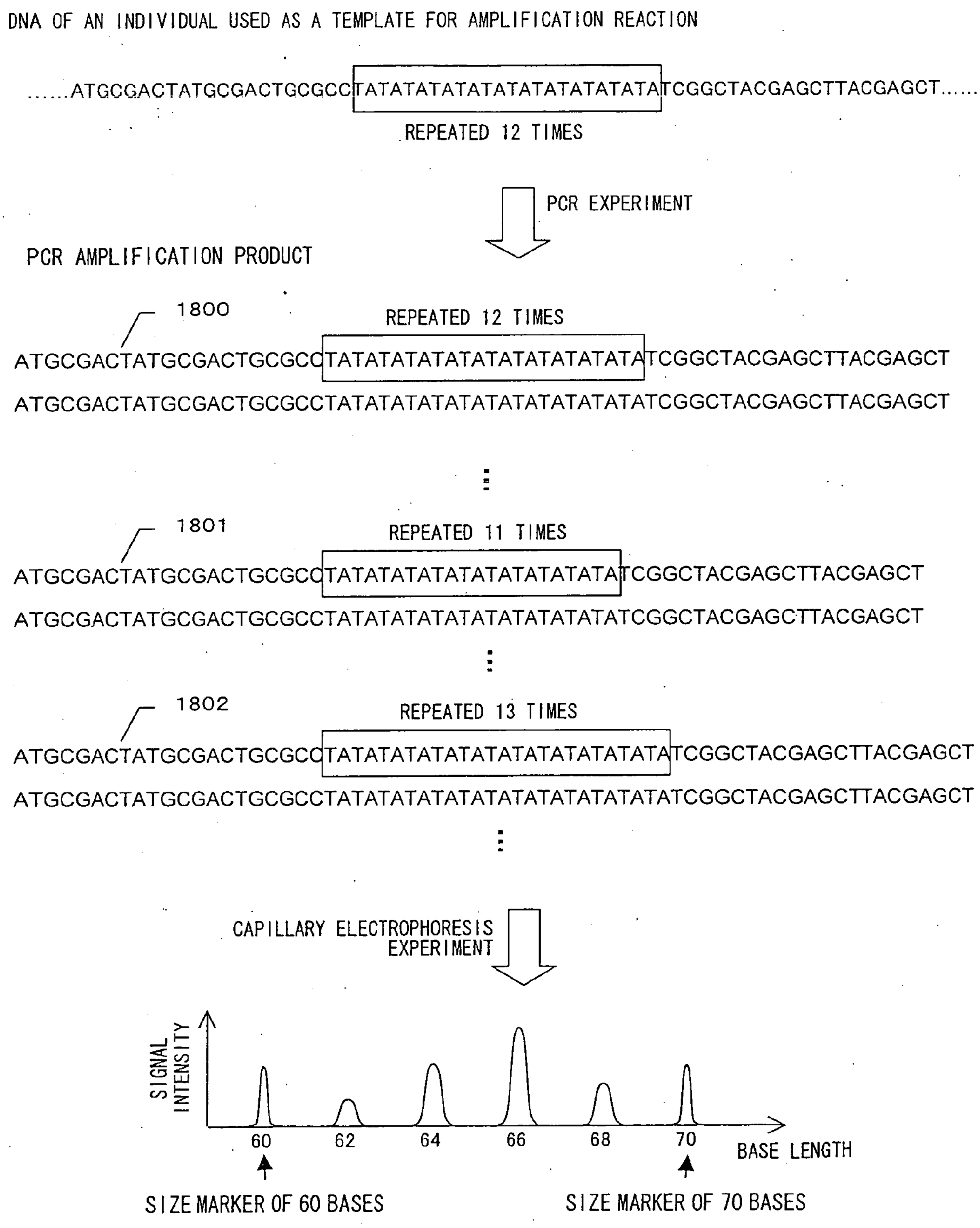
DNA OF AN INDIVIDUAL USED AS A TEMPLATE FOR AMPLIFICATION REACTION
......ATGCGACTATGCGACTGCGCCTATATATATATATATATATATATATCGGCTACGAGCTTACGAGCT......
REPEATED 12 TIMES
PCR EXPERIMENT
PCR AMPLIFICATION PRODUCT
1800
REPEATED 12 TIMES
ATGCGACTATGCGACTGCGCCTATATATATATATATATATATATATCGGCTACGAGCTTACGAGCT
ATGCGACTATGCGACTGCGCCTATATATATATATATATATATATATCGGCTACGAGCTTACGAGCT
1801
REPEATED 11 TIMES
ATGCGACTATGCGACTGCGCCTATATATATATATATATATATATCGGCTACGAGCTTACGAGCT
ATGCGACTATGCGACTGCGCCTATATATATATATATATATATATCGGCTACGAGCTTACGAGCT
1802
REPEATED 13 TIMES
ATGCGACTATGCGACTGCGCCTATATATATATATATATATATATATATCGGCTACGAGCTTACGAGCT
ATGCGACTATGCGACTGCGCCTATATATATATATATATATATATATATCGGCTACGAGCTTACGAGCT
CAPILLARY ELECTROPHORESIS EXPERIMENT
SIGNAL INTENSITY
60
62
64
66
68
70
BASE LENGTH
SIZE MARKER OF 60 BASES
SIZE MARKER OF 70 BASES

FIG. 19

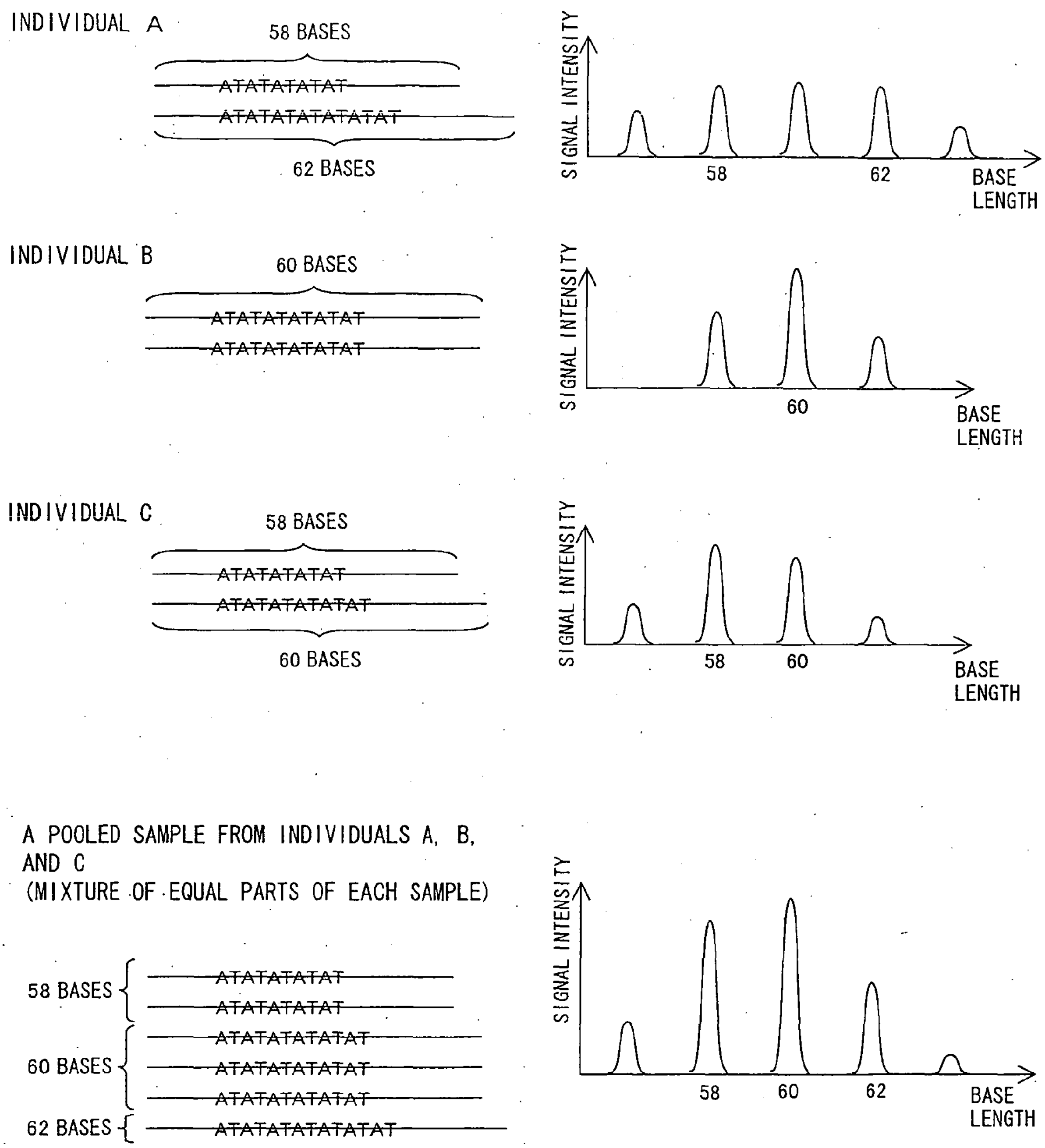


| ALLELE | 56 | 58 | 60 | 62 | 64 |
|---|---|---|---|---|---|
| PERCENTAGE | 10.3% | 31.4% | 36.3% | 18.5% | 3.5% |
| FREQUENCY | 0.6 | 1.9 | 2.2 | 1.1 | 0.2 |

FIG. 20

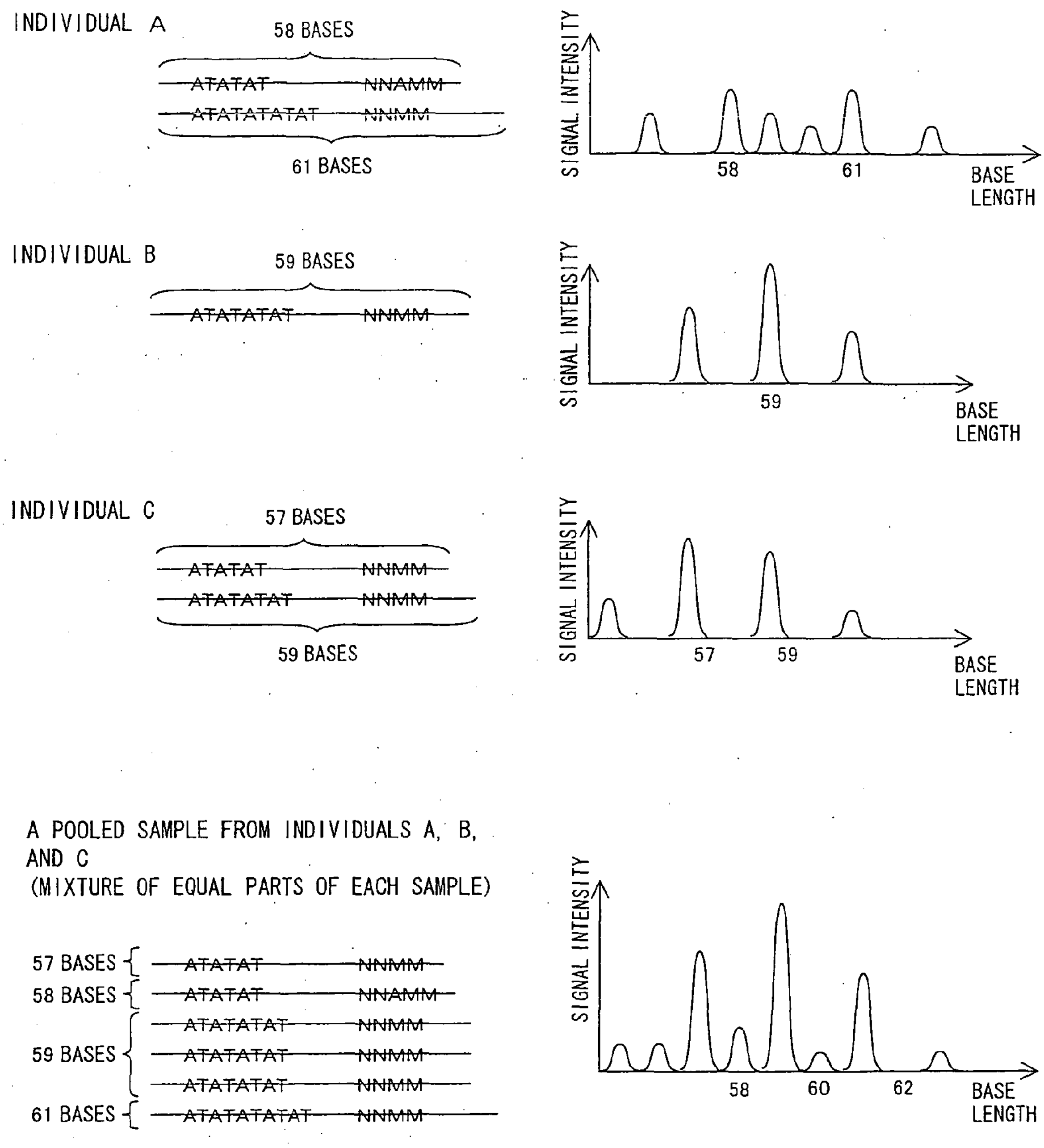


| ALLELE | 55 | 56 | 57 | 58 | 59 | 60 | 61 | 63 |
|---|---|---|---|---|---|---|---|---|
| PERCENTAGE | 4.8% | 4.8% | 22.8% | 8.4% | 33.5% | 3.5% | 18.8% | 3.5% |
| FREQUENCY | 0.3 | 0.3 | 1.4 | 0.5 | 2.0 | 0.2 | 1.1 | 0.2 |